# EUROPÄISCHE PATENTSCHRIFT

(11) **EP 2 694 535 B1**
(45) Veröffentlichungstag und Bekanntmachung des Hinweises auf die Patenterteilung: **07.08.2019**
(21) Anmeldenummer: 12732951.4
(22) Anmeldetag: 24.03.2012
(51) Int. Cl.: C07K 14/32, A01N 63/00, C12R 1/07

(54) **MITTEL ZUR BEHANDLUNG VON MIKROBIELLEN ERKRANKUNGEN BEI KULTURPFLANZEN**
MEANS FOR TREATING MICROBIAL DISEASES IN CULTIVATED PLANTS
AGENT POUR LE TRAITEMENT DE MALADIES BACTÉRIENNES DE PLANTES CULTIVÉES

(30) Priorität: 01.04.2011 DE 102011015803
(43) Veröffentlichungstag der Anmeldung: 12.02.2014
(73) Patentinhaber: Abitep GmbH, 12489 Berlin (DE)
(72) Erfinder: BORRISS, Rainer, 12489 Berlin (DE)
(74) Vertreter: Baumbach, Friedrich
(86) Internationale Anmeldenummer: PCT/DE2012/000326
(87) Internationale Veröffentlichungsnummer: WO 2012/130221

(56) Entgegenhaltungen:
- EP-A1- 2 179 652
- CHEN X H ET AL: "Genome analysis of Bacillus amyloliquefaciens FZB42 reveals its potential for biocontrol of plant pathogens", JOURNAL OF BIOTECHNOLOGY, ELSEVIER SCIENCE PUBLISHERS, AMSTERDAM, NL, Bd. 140, Nr. 1-2, 10. März 2009 (2009-03-10), Seiten 27-37, XP025969900, ISSN: 0168-1656, DOI: 10.1016/J.JBIOTEC.2008.10.011 [gefunden am 2008-11-12] in der Anmeldung erwähnt
- CHEN X H ET AL: "Difficidin and bacilysin produced by plant-associated Bacillus amyloliquefaciens are efficient in controlling fire blight disease", JOURNAL OF BIOTECHNOLOGY, ELSEVIER SCIENCE PUBLISHERS, AMSTERDAM, NL, Bd. 140, Nr. 1-2, 10. März 2009 (2009-03-10), Seiten 38-44, XP025969901, ISSN: 0168-1656, DOI: 10.1016/J.JBIOTEC.2008.10.015 [gefunden am 2008-11-18] in der Anmeldung erwähnt
- S. COMPANT ET AL: "Use of Plant Growth-Promoting Bacteria for Biocontrol of Plant Diseases: Principles, Mechanisms of Action, and Future Prospects", APPLIED AND ENVIRONMENTAL MICROBIOLOGY, Bd. 71, Nr. 9, 1. September 2005 (2005-09-01), Seiten 4951-4959, XP055043453, ISSN: 0099-2240, DOI: 10.1128/AEM.71.9.4951-4959.2005

## Beschreibung

### Einleitung

Die Erfindung betrifft ein biologisches Mittel zur Behandlung von pilzlichen Erkrankungen bei Kulturpflanzen auf der Basis eines Bacillus amyloliquefaciens subsp. plantarum-Stammes sowie seine Verwendung.

Anwendungsgebiete der Erfindung sind der Pflanzenschutz, die Biotechnologie und die Landwirtschaft.

### Stand der Technik

Die umweltfreundliche Kontrolle von mikrobiell verursachten Erkrankungen stellt eine dringende Notwendigkeit dar, um den zunehmenden Nahrungsbedarf einer wachsenden Weltbevölkerung zu decken. Heutige Praktiken beruhen größtenteils auf der genetischen Resistenz der Wirtspflanze, einem abgestimmtem Management von Pflanze und Umwelt und dem Einsatz synthetischer Pestizide, die nach andauernder Anwendung ihre Wirksamkeit einbüßen und häufig einen ungünstigen Einfluss auf die Umwelt ausüben. Die Einführung von biologischen Pflanzenschutzmitteln ("Biopestizide") stellt eine attraktive Alternative zu den bisher überwiegend eingesetzten chemischen Pflanzenschutzmitteln dar. Biopestizide werden von der US Umweltschutzagentur EPA (Environmental Protection Agency) als Pflanzenschutzmittel aus "natürlichem Material" (Tiere, Pflanzen, Bakterien und bestimmte Mineralien) definiert. Dabei haben sich biologische Pflanzenschutzmittel aus Bakterien und Pilzen als besonders wirksam erwiesen. Z.B. hat sich die Entwicklung von Bioformulierungen von so genannten "Pflanzenwachstumsfördernden Bakterien" ("plant growth promoting bacteria", PGPR) mit Biokontrolleigenschaften als besonders viel versprechend gezeigt. Diese Bakterien vermögen nicht nur das Pflanzenwachstum zu fördern, sondern unterdrücken gleichzeitig auch die phytopathogene Mikroflora und beugen der Erkrankung durch pilzliche und bakterielle Erreger durch Induktion der systemischen Resistenz vor. Vertreter der Gattung *Pseudomonas*, z.B. *P. fluorescens*, haben sich in der Vergangenheit als effiziente Kolonisatoren der pflanzlichen Wurzelzone, die gleichzeitig die phytopathogene Begleitflora wirkungsvoll unterdrücken, erwiesen. Die Anwendung der aus diesen Bakterien hergestellten industriellen Produkte ist jedoch durch die begrenzte Haltbarkeit und Lagerfähigkeit der vegetativen Bakterienzellen eingeschränkt. Eine erfolgreiche Alternative stellt der Einsatz von ausgewählten sporenbildenden Bakterien, z. B. Repräsentanten der Gattung *Bacillus*, dar. Sporen sind Dauerformen der Bakterienzelle, die über eine, chemischen Präparaten vergleichbare oder überlegene, Haltbarkeit verfügen. Sie widerstehen extremen Umwelteinflüssen (Temperatur, UV-Strahlung, chemischen Agentien) und können jahrelang ohne Aktivitätsverlust gelagert werden. Bereits heute sind Bioformulierungen von Repräsentanten der Gattung *Bacillus*, die am häufigsten eingesetzten biologischen Pflanzenschutzmittel (http://www.agraquest.com/). Neben den bekannten Produzenten von Insektiziden, *Bacillus thuringiensis* und *Bacillus kurstakii*, sind es Stämme aus dem Verwandtschaftskreis von *Bacillus subtilis*, die die meiste Anwendung auf diesem Gebiet erfahren haben. Allerdings ist ihre erfolgreiche Anwendung durch die unzureichende Kenntnis der molekularen und metabolischen Prinzipien, die ihrer - für die Pflanzen vorteilhaften - Wirkung zugrunde liegen, bisher noch stark eingeschränkt. Beispielsweise wurde in PCT WO 00/29426 die antagonistische Wirkung des Bacillus subtilis Stammes AQ713 auf die Bildung der Lipopeptide IturinA, Plipastatin, Surfactin und einer als "Agrastatin" bezeichneten Gruppe von zyklischen Lipopeptiden, die ebenso wie Plipastatin den Fengycinen entsprechen , zurückgeführt. Mit Ausnahme von Surfactin, das eine schwache antibakterielle Wirkung aufweist, wirken diese Sekundärmetabolite jedoch nur gegen phytopathogene Pilze (8). Aktuelle Untersuchungen ergaben, dass Vertreter einer distinkten taxonomischen Gruppe - *Bacillus amyloliquefaciens* subsp. *plantarum -* sich durch ein hohes Potential zur Bildung antifungaler und antibakterieller Metaboliten - auszeichnen (1). Repräsentanten dieser Gruppe sind für ihre pflanzenwachstumsfördernden Eigenschaften und für ihre Fähigkeit zur Suppression phytopathogener Mikroorganismen in der pflanzlichen Wurzelzone bekannt (1). Beispiele für antifungale Wirkstoffe, die durch Repräsentanten dieser taxonomischen Gruppe gebildet werden, sind iturinartige Lipopeptide (Bacillomycin D oder Iturin A) und Fengycin. Weitere Produkte mit biologischer Bedeutung, die auf nicht-ribosomalen Wege hergestellt werden, sind das Eisensiderophor Bacillibactin und das antimikrobielle Dipeptid Bacilysin. Darüber hinaus sind Repräsentanten der "plantarum" Gruppe für die Synthese der antibakteriellen Polyketide Bacillaen, Makrolactin und Difficidin bekannt. Kürzlich wurde eine weitere Verbindung mit schwacher antimikrobieller Wirkung beschrieben, Plantazolicin. Anders als die vorher benannten Lipopeptide und Polyketide wird Plantazolicin durch konventionelle, ribosomale Synthese hergestellt (4). Weiterhin induzieren diese Bakterien gezielte Abwehrreaktionen der Pflanze gegen biotischen und abiotischen Stress, die als "Induzierte systemische Resistenz", ISR, bezeichnet werden und produzieren das pflanzliche Wachstumshormon 2,3-Indolyl-Essigsäure (9). Der Typstamm für die "*plantarum*" Subspecies, das Rhizobakterium FZB42 (=DSM 23117), weist pflanzenwachstumsfördernde und Phytopathogen-supprimierende Eigenschaften auf (2-12) und wird als Bodenhilfsstoff "Rhizovital®" kommerziell im Agrar- und Gartenbereich eingesetzt (http://www.abitep.de/). Voraussetzung für eine effiziente Wirkung des Wurzelbakteriums ist eine effiziente und dauerhafte Besiedlung der pflanzlichen Wurzelzone. Hier weist FZB42, ebenso wie andere zur Förderung des Pflanzenwachstums und zur biologischen Kontrolle phytopathogener Mikroorganismen bisher eingesetzte Bacillus-Stämme (vgl. Patentschriften: US 6,960,342 (2005), US 6,015,553 (2000), US0265292 A1 (2004), US 6,524,998 B1 (2003), EP 1 449 914 A1 (2002), WO 00/29426 (2000)) Nachteile gegenüber wurzelbesiedelnden Gram-negativen Bakterien, z.B. *Pseudomonas*, auf. Es wurde festgestellt, dass wurzelbesiedelnde Bacillen nur relativ kurze Zeit - nicht länger als sechs Wochen - an den Pflanzenwurzeln nachweisbar waren. Es wird vermutet, dass dieser Effekt auf fehlender Kompetitivität gegenüber anderen Vertretern der mikrobiellen Wurzelflora beruht. Somit kann eine lang andauernde und stabile Wirkung auf das Pflanzenwachstum durch die augenblicklich eingesetzten Bacillusbasierenden Bioformulierungen nicht erwartet werden und die Wirkung dieser Formulierungen ist durch die unzureichende Kenntnis ihrer wirksamen Komponenten eingeschränkt.

### Ziel und Aufgabe der Erfindung

Die Erfindung hat das Ziel, ein umweltverträgliches, lagerfähiges und lang andauernd wirksames Mittel gegen phytopathogene Mikroorganismen, insbesondere Pilze, auf der Grundlage definierter biologischer Faktoren zu entwickeln.

Aufgabe der Erfindung ist es, effektive Bacillus-Stämme, die der Species *Bacillus amyloliquefaciens* subsp. *plantarum* angehören und ein lang andauernd wirksames, antagonistisches Potential aufweisen, für die Entwicklung eines natürlichen, gesundheitlich unbedenklichen Mittels zur Vorbeugung und Behandlung von mikrobiellen Erkrankungen, insbesondere pilzlichen Infektionen, z. B. *Rhizoctonia solani* an Kartoffeln und anderen Kulturpflanzen, einzusetzen.

### Wesen der Erfindung

Die Aufgabe wird gemäß den Ansprüchen 1-11 gelöst; die Unteransprüche sind Vorzugsvarianten.

Bei der Suche nach Mikroorganismen mit verbesserter antagonistischer Aktivität gegen phytopathogene Mikroorganismen wurde gefunden, dass Mitglieder der Subspecies *Bacillus amyloliquefaciens plantarum* eine ausgezeichnete Wirkung gegen Erreger pilzlicher und anderer mikrobieller und viraler Krankheiten an Kulturpflanzen aufweisen. Unter diesen Stämmen hat der Erfinder der vorliegenden Erfindung den Stamm ABI01 als Vorzugsvariante gewählt, der in seiner Persistenz in der pflanzlichen Rhizosphäre und in seiner antifungalen Leistung den Typstamm FZB42 übertrifft.

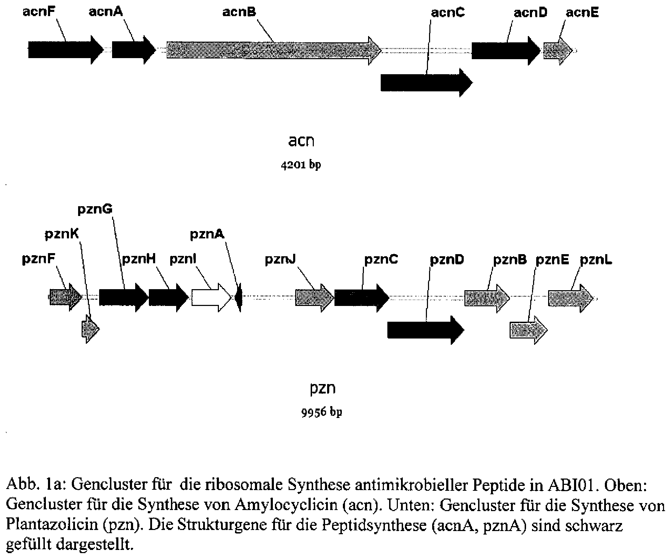

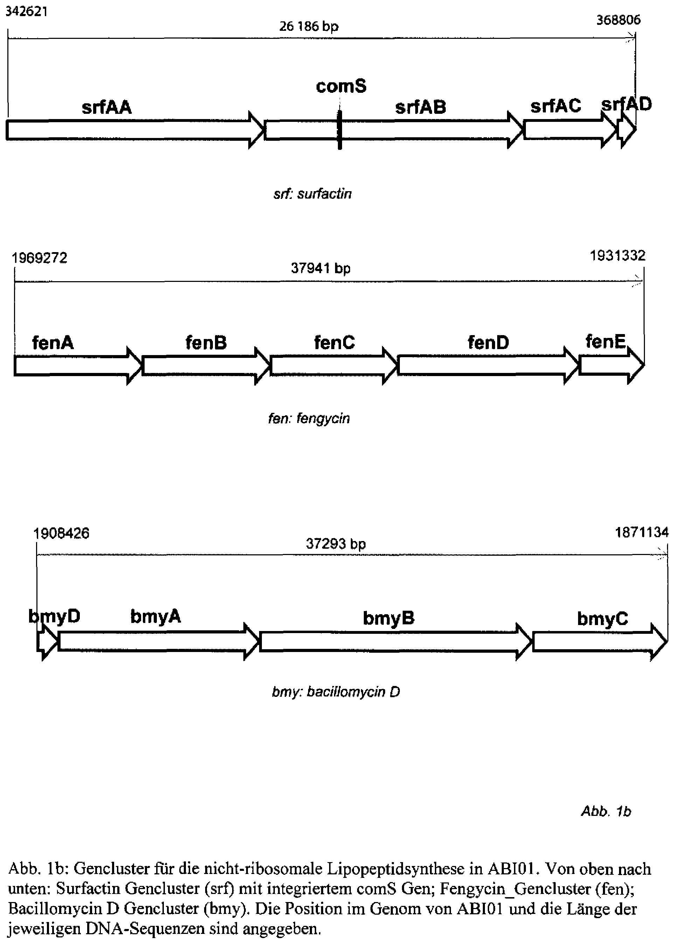

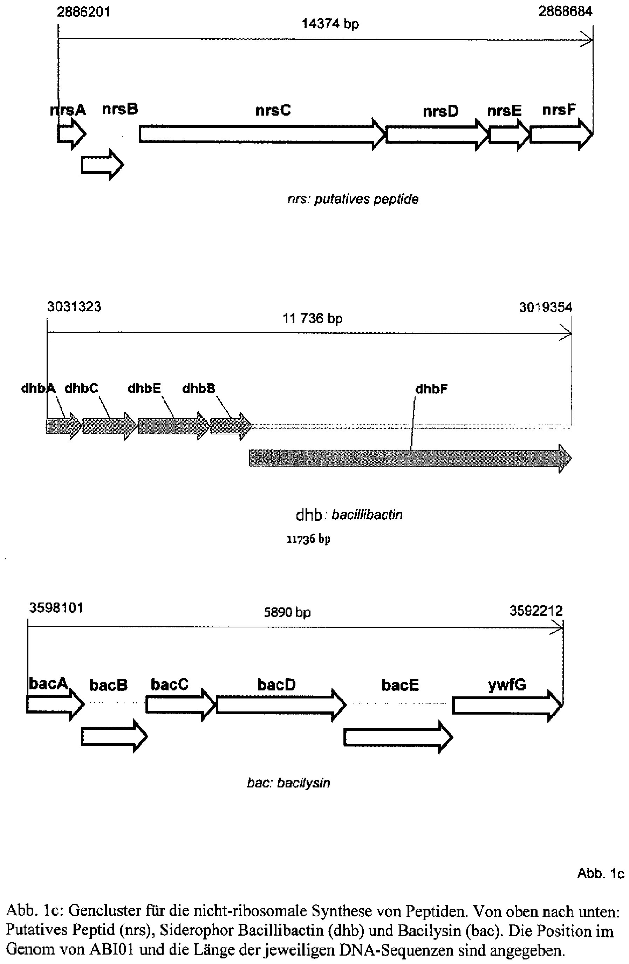

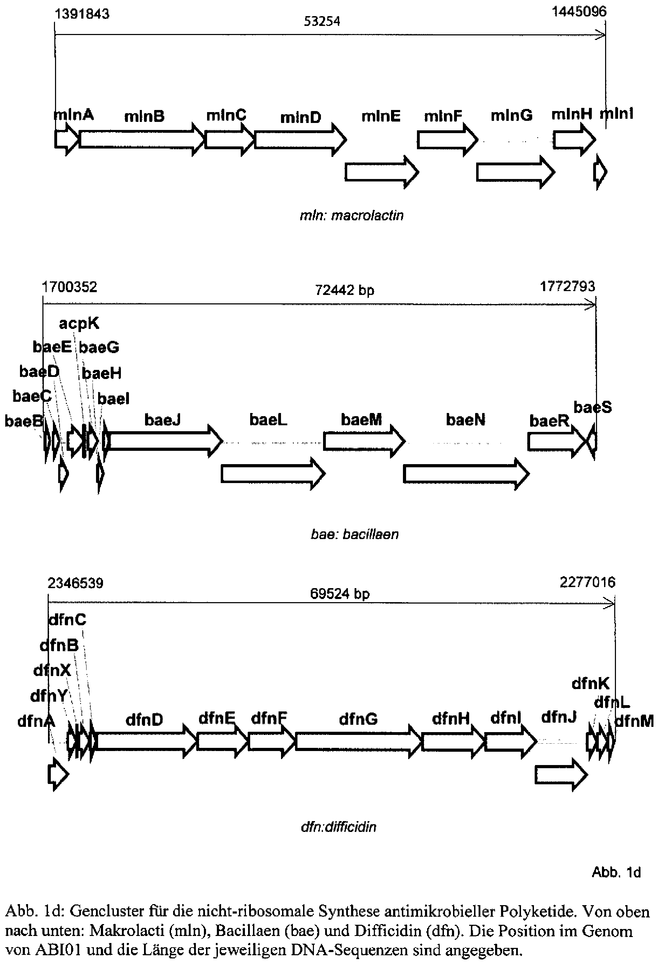

Der pflanzenassozierte Bacillus-Stamm ABI01 der vorliegenden Erfindung ist ein Vertreter der taxonomischen Gruppe *Bacillus amyloliquefaciens* subsp. *plantarum.* Repräsentanten dieser Gruppe sind für ihre pflanzenwachstumsfördernden Eigenschaften und für ihre Fähigkeit zur Suppression phytopathogener Mikroorganismen in der pflanzlichen Wurzelzone bekannt (1). Darüber hinaus induzieren sie gezielte Abwehrreaktionen der Pflanze gegen biotischen und abiotischen Stress, die als "Induzierte systemische Resistenz", ISR, bezeichnet wird und produzieren das pflanzliche Wachstumshormon 2,3-Indolyl-Essigsäure (9). Durch die gezielte Anreicherung von Vertretern dieser taxonomischen Gruppe aus der pflanzlichen Wurzelzone konnte der Stamm ABI01 gewonnen werden, der überraschenderweise in seiner antifungalen Wirksamkeit andere Vertreter dieser Gruppe, z.B. den Typstamm FZB42, deutlich übertrifft. Die komplette Genomsequenz von ABI01 wurde bestimmt und ist Bestandteil dieser Patentanmeldung. Die Analyse der kompletten Genomsequenz ergab die Anwesenheit von elf Genclustern, die in die Synthese von antimikrobiellen Verbindungen, darunter einem hypothetischen Produkt, eingebunden sind (Abb. 1-d, Tabelle 1). Es wurden insgesamt 10 Sekundärmetabolite mit antifungaler, antibakterieller und antiviraler Wirksamkeit in der Kulturlösung bzw. Oberflächenzellextrakten von ABI01 direkt nachgewiesen. Isoformen von Bacillomycin D mit unterschiedlicher Länge des Fettsäurerestes wurden als Hauptkomponenten der antagonistischen Wirkung gegenüber phytopathogenen Pilzen identifiziert. Das hier erstmalig beschriebene, cyclische Peptid Amylocyclicin supprimiert das Wachstum Gram-positiver Bakterien. Das Polyketid Difficidin wirkt gegen Gram-positive und Gram-negative Bakterien; Bacilysin hat eine generelle antimikrobielle Wirkung. In vergleichenden Feldversuchen erwies ABI01 sich gegenüber anderen eingesetzten Bio- und Chemofungiziden bei der Suppression von Rhizoctonia solani an Kartoffeln als überlegen und zeigte eine höhere Wirksamkeit als FZB42 und das Chemopestizid Cuprozin (Ausführungsbeispiel 7). Gemäß der vorliegenden Erfindung wird der antifungal wirkende ABI01 (Zugangsnummer: DSM 10-1092) zur Verfügung gestellt, der als *Bacillus amyloliquefaciens* subsp. *plantarum* klassifiziert wurde.

### Nachfolgend wird die vorliegende Erfindung ausführlich beschrieben

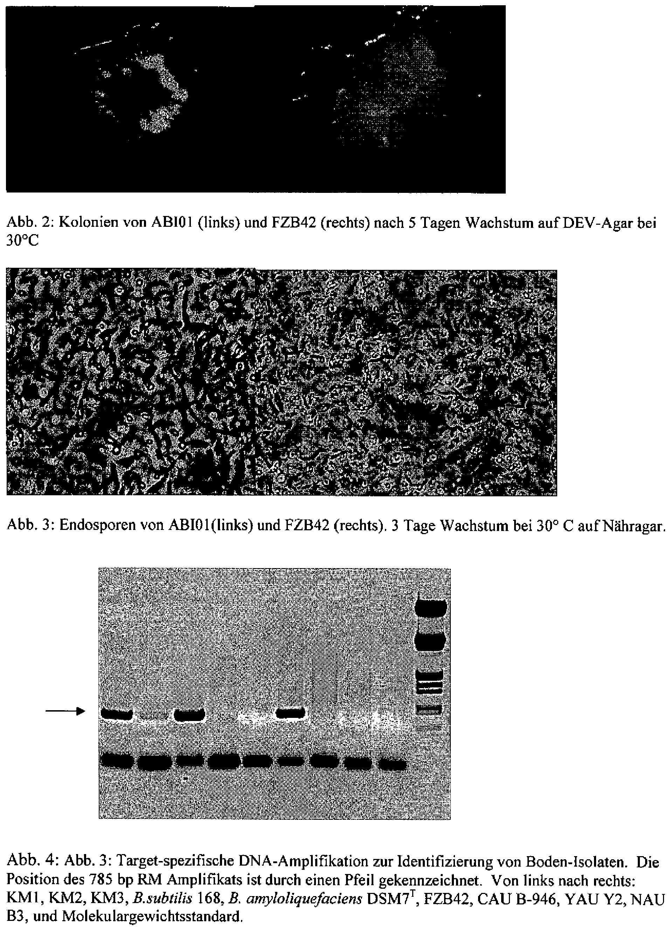

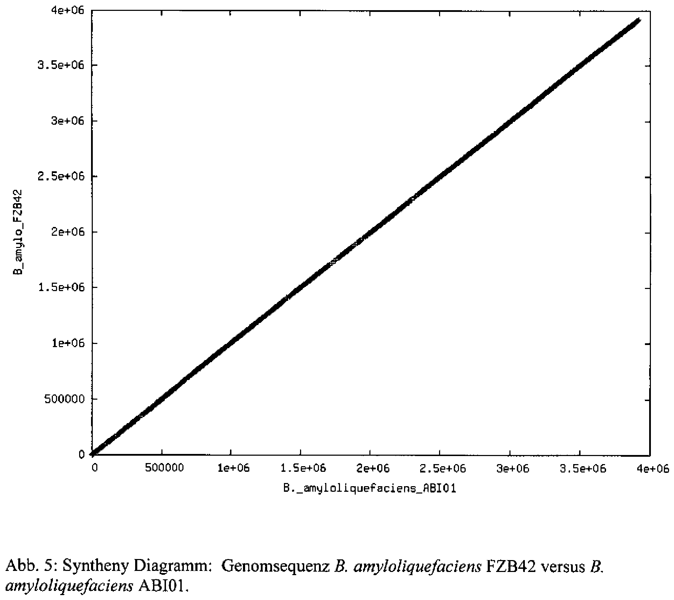

ABI01 ist ein Vertreter der taxonomischen Gruppe *Bacillus amyloliquefaciens* subsp. *plantarum* (1). Der Stamm wurde durch die gezielte Anreicherung von Vertretern dieser taxonomischen Gruppe aus der pflanzlichen Wurzelzone gemäß Ausführungsbeispiel 1 erhalten. Kolonien des Endosporen-bildenden Bakteriums (Abb. 3) wiesen die Fähigkeit zur Laktoseverwertung und zur Hydrolyse von Hydroxyethyl-Cellulose auf, Merkmale die für die Differenzierung dieser taxonomischen Gruppe von Bedeutung sind. Der Stamm ähnelt in seiner Kolonieform dem Stamm FZB42, ist jedoch nach 5-tägigem Wachstum auf DEV-Agar von diesem Stamm deutlich unterscheidbar (Abb. 2). Weitere phänotypische Merkmale des Stammes ABI01 sind in der Tabelle 3 zusammengefasst. Durch den Einsatz Sequenz-spezifischer Primer gelang die Amplifikation von DNA-Fragmenten, die für die "*plantarum*" Gruppe und speziell für den Typstamm FZB42 charakteristisch sind (1, Abb. 4). Es handelt sich um Sequenzen für ein Gen des Restriktions - und Modifikationssystems (RM), eines Gens für die nicht-ribosomale Synthese eines unbekannten Peptids (NRSP) und des Bacteriocins "Plantazolicin" (pzn). Damit wurde der Nachweis erbracht, dass die erhaltenen Isolate dem taxonomischen Umfeld von "*plantarum*" zuzuordnen sind und in enger Verwandtschaft zum Typstamm FZB42 stehen. Diese Einordnung wurde durch die Sequenzierung der kompletten Genomsequenz von ABI01 (3,9 Mbp) bestätigt (Tabelle 4). Die ABI01 Genomsequenz weist nur geringe Unterschiede zur Genomsequenz des Typstammes FZB42 auf (Abb. 5, Ausführungsbeispiel 3), 64 Stellen in der Genomsequenz weisen Unterschiede zu FZB42 auf (Tabelle 6). Das ABI01 Genom enthält alle Gencluster, die für die Synthese von Metaboliten mit antagonistischer Wirkung gegen mikrobielle und virale Krankheitserreger bei Kulturpflanzen von Bedeutung sind und von anderen Vertretern der taxonomischen Gruppe *Bacillus amyloliquefaciens* subsp. *plantarum*, z.B. FZB42, bekannt sind (Tabelle 1, Abb. 1a-d). Die Bildung dieser Sekundärmetaboliten verursacht beispielsweise im Fall von Bacillomycin D und Fengycin eine Wachstumshemmung phytopathogener Pilze, z. B. von *Rhizoctonia solani* und *Fusarium oxysporum.* Antibakterielle Wirkung haben die Polyketide Difficin, Bacillaen und Makrolaktin, sowie das Dipeptid Bacilysin. Eine antivirale Wirkung wird für das oberflächenaktive Surfactin angenommen. Das Siderophor Bacillibactin wird unter Eisenmangelbedingungen induziert und trägt zur besseren Eisenversorgung der betreffenden Pflanzen bei. Die Expression der hier genannten Sekundärmetaboliten, sowie des hochmodifizierten Peptids Plantazolicin konnte direkt durch HPLC und MALDI TOF MS der Kulturfiltrate bzw. durch Messung der Oberflächenextrakte nachgewiesen werden (Ausführungsbeispiel 5). Damit wurde der Nachweis erbracht, dass die erhaltenen Isolate dem taxonomischen Umfeld von "*plantarum*" zuzuordnen sind und in enger verwandtschaftlicher Beziehung zu dem Typ-Stamm FZB42 stehen. Das ist überraschend, da bisher angenommen wurde, dass ein Nachweis von Vertretern der *Bacillus*-Gruppe im Bereich der pflanzlichen Wurzelzone nur maximal sechs Wochen nach Applikation dieser Bakterien möglich ist. Somit weisen die erhaltenen Isolate eine, gegenüber FZB42 und anderen bisher eingesetzten *Bacillus*-Stämmen, erhöhte Fähigkeit zur Überdauerung (Persistenz) im Bereich der pflanzlichen Wurzelzone auf und sind für den Einsatz bei lang andauernden Kulturen pflanzlicher Nutzpflanzen von besonderem Vorteil. Vergleichende Feldversuche mit Kartoffeln zeigten, dass eine Beize der Kartoffelknollen mit ABI01 zu einer gegenüber FZB42 verbesserten phytosanitären Wirkung führt und die durch den Pilz *Rhizoctonia solani* verursachte Wurzeltöterkrankheit ("black scurf") stärker zurückdrängt als bei Einsatz alternativer Bio- und Chemofungizide.

Zur Ausbildung von Sporen für die Massenproduktion wird der *Bacillus amyloliquefaciens plantarum* ABI01 der vorliegenden Erfindung unter einer geeigneten Bedingung kultiviert (Ausführungsbeispiel 6). Das Produkt kann als flüssige Sporensuspension, als Trockenbeize oder als Kombination von Bacillus-Sporen und einem Sekundärmetabolitenkonzentrat unter Zusatz sowohl als Gießpräparat für die Bodenbehandlung oder als Sprühpräparat für die Blattbehandlung eingesetzt werden.

Die verschiedenen Anwendungsmöglichkeiten sind ebenfalls Gegenstand dieser Erfindung. Bevorzugt enthalten diese Präparate mindestens 1 x 10¹⁰ Sporen pro Gramm bzw. pro Milliliter.

Flüssig-Konzentrate werden aus einer Bacillus amyloliquefaciens subsp. plantarum Kultur gewonnen, welche eine Lipopeptidkonzentration von mindestens 1 g/L enthält. Eingesetzt werden auch Flüssig-Konzentrate gewonnen aus einer Bacillus amyloliquefaciens subsp. plantarum Kultur mit einer Bacillomycin D Konzentration von mindestens 1 g/L.

Nachfolgend wird die vorliegende Erfindung spezieller durch Beispiele beschrieben. Die folgenden Beispiele sind jedoch nur zur Erläuterung angegeben und daher ist die vorliegende Erfindung nicht durch sie oder auf sie beschränkt.

### Ausführungsbeispiele

### Beispiel 1: Anreicherung und Isolierung von Bacillus amyloliquefaciens plantarum

Es wurde eine spezifische Methode zur Isolierung von pflanzen-assoziierten *Bacillus amyloliquefaciens plantarum* Stämmen entwickelt:
1. 2,5 g einer Erdprobe, die aus der pflanzlichen Rhizosphäre entnommen worden war, wurden in 25 ml destilliertem Wasser für 2 h bei Raumtemperatur unter Schütteln resuspendiert.
2. Die Suspension wurde eine Stunde gekocht.
3. 10 ml der Suspension wurden zu 40 ml in einem flüssigem Minimalmedium mit Lactose (0,1%) als einziger C-Quelle zugegeben und für mehrere Tage bei 30°C inkubiert, bis bei der mikroskopischen Kontrolle stäbchenförmige Zellen sichtbar wurden.
4. Verdünnung der Proben von 10⁻¹ bis 10⁻⁵ und Ausplattierung auf 1,5% Minimal-Agar mit Laktose als einziger C-Quelle und Zusatz von 0.1% AZCL HE (Azure dye angefärbte Hydroxyl Ethyl Cellulose). Inkubation bei 30°C für drei bis fünf Tage. Kolonien, die eine Hydrolyse der Zellulose aufwiesen und in Ihrer Koloniemorphologie dem Typstamm FZB42 (rau, flach, dendritisch, weiß bis durchscheinend) ähnelten (Abb.1), wurden ausgewählt.
5. Chromosomale DNA wurde von diesen Kolonien nach der Phenol-Standardmethode (13) isoliert und für die Polymerase-Ketten Reaktion (PCR) mit den sequenzspezifischen Primern PRBrm5215 tgatggagtaaataataaggctgg, und PRBrm6000 aatacatctaaagttgcatccacc eingesetzt. Diese Reaktion dient dem Nachweis eines, für FZB42 spezifischen 785 bp-Fragmentes des Restriktions- und Modifikationssystem (RM). Darüber hinaus kommen die nachfolgenden Sequenz-spezifische Primerpaare zum Einsatz: PRBnrs3104 tggagaaatatcactgaacaatgc und PRBnrs3943 acgtttagtttcagttctttcacc für die Amplifikation des NRSP-Gen-Fragmentes und PRBptn6179 gatagaagtattagcctggaagca und PRBptn7000 tggaggaggtaacaattatgactc für die Amplifikation eines Gens aus dem Plantazolicin-Synthese Gencluster.

Ein Beispiel für die Anwendung dieser Methode ist nachfolgend gegeben:
Der Versuch wurde in einem Gewächshaus in Lianpengwei village, Dounan town, Chenggong county, Kunming, China, im März 2010 durchgeführt.

Fünf Monate nach Anwendung einer kommerziellen Sporensuspension von FZB42 (Rhizovital®) zu einer Kultur von Löwenmäulchen (*Antirhinum major*) wurden entsprechende Bodenproben entnommen und wie oben geschildert behandelt. Die erhaltenen Kolonien wiesen ein für FZB42 typisches Aussehen auf (Abb. 2) und waren zur Nutzung von Laktose als einziger C-Quelle und zur Hydrolyse von HE Cellulose fähig. Die Zellen bildeten nach dreitägiger Kultur auf Nähragar bei 30°C ovale Endosporen aus. Die Sporenmutterzellen waren nicht angeschwollen; die Lage der Sporen war zentral oder vereinzelt distal (Abb. 3). Die mit chromosomaler DNA und sequenzspezifischen Primern (s.o.) durchgeführte PCR erbrachte die Amplifikation der für die BAP-Gruppe typischen Genfragmente einschließlich des für FZB42 charakteristischen Fragments des RM-Systems (Abb.4). Die Ergebnisse sind in der Tabelle 2 zusammengefasst.

Der Stamm KM1-1 (ABI01) wurde für die weiteren Untersuchungen ausgewählt. Er wurde unter der Nummer DSM 10-1092 in der deutschen Sammlung von Mikroorganismen und Zellkulturen GmbH, DSMZ, deponiert und seine taxonomische Zugehörigkeit zu BAP durch Untersuchungen des DSMZ bestätigt (siehe Ausführungsbeispiel 2).

### Beispiel 2: Phänotypische und genotypische Charakterisierung von Bacillus amyloliquefaciens plantarum ABI01 (DSM 10-1092)

Die Merkmale zur taxonomischen Identifizierung von ABI01 (DSM 10-1092) sind in der Tabelle 2 zusammengefasst und bestätigen die Einordnung als B. *amyloliquefaciens* subsp. *plantarum.*

Weiterhin wurde eine Untersuchung des zellulären Fettsäureprofils von ABI01 durchgeführt, dass die größte Ähnlichkeit zu *B. subtilis* (Index der Ähnlichkeit: 0,631) und *B. amyloliquefaciens* (Index der Ähnlichkeit: 0,614) ergab. Ein gleichfalls durchgeführter Vergleich von ABI01 und FZB42 im Rahmen einer Riboprinteranalyse (Restriktionsenzym: EcoRI) ergab, dass beide Stämme einen Ähnlichkeitsindex von ≥ 0,93 aufweisen und somit der gleichen RiboGruppe, ECORI 198-1901-S4, angehören. Stämme, die der gleichen RiboGruppe angehören können durch das Riboprint-System nicht weiter differenziert werden und werden nach Aussage des DSMZ als identisch betrachtet. Die Partialsequenz der 16SrRNA wies nach Auskunft des DSMZ eine Übereinstimmung von ≥99,8% zu Sequenzen von *B. subtilis* und B. *amyloliquefaciens* auf und ist somit für eine weitergehende taxonomische Differenzierung des Stammes nicht ausreichend (vgl. Borriss et al. 2010).

### Referenz

Borriss, R. et al. Relationship of Bacillus amyloliquefaciens clades associated with strains DSM7T and FZB42; a proposal for Bacillus amyloliquefaciens subsp. amyloliquefaciens subsp. nov. and Bacillus amyloliquefaciens subsp. plantarum subsp. nov. based on their discriminating complete genome sequences. IJSEM, published ahead of print Sept 3, 2010

### Beispiel 3: Genomsequenz von ABI01

Die Sequenzierung von chromosomaler DNA des Stammes ABi01 wurde mittels eines Roche/454 (GS-FLX Titanium)- Sequenziergerätes durchgeführt. Es wurden insgesamt ca. 330.000 Sequenzen (118 Mb) mit einer durchschnittlichen Leseweite von 350 bp erzeugt, die zu 25 Contigs zusammengefasst werden konnten. Die Sequenzen wiesen eine hohe Übereinstimmung mit dem Genom des pflanzenwachstumsfördernden Bakterium *Bacillus amyloliquefaciens* subsp. *plantarum* FZB42 auf (Abb. 5). 99,34% der Referenzsequenz von FZB42 wurden durch die Sequenzierung abgedeckt. Die vollständige Genomsequenz von ABI01 wurde durch Vergleich mit FZB42 assembliert und annotiert. Die wichtigsten Genomdaten von ABI01 im Vergleich zu FZB42 sind in der Tabelle 4 aufgeführt. Insgesamt wurden 35 neue CDS in ABI01, die nicht in der FZB42 Genomsequenz annotiert worden waren, identifiziert (Tabelle 5). Insgesamt wurden 283 Stellen, die Sequenzunterschiede (SNPs) zu FZB42 aufwiesen, davon 65 mit hoher statistischer Sicherheit, in der ABI01 Genomsequenz identifiziert (Tabelle 6). Von diesen Veränderungen waren insgesamt 32 CDS betroffen. Bei neun von diesen 32 Genen handelt es sich vermutlich um Sequenzierungsfehler im Genom von FZB42. Bei zwei weiteren Genen handelte es sich um "stille" Mutationen, die keine weiteren Auswirkungen auf die Aminosäuresequenz hatten. Somit weisen in dem Genom von ABI01 wenigstens 19 CDS gegenüber FZB42 Unterschiede in der Aminosäuresequenz auf und die Genome von ABI01 und FZB42 sind nicht identisch.

### Beispiel 4: Identifizierung des antibakteriellen, cyclischen Peptids, Amylocyclizin

Als antibakteriell wirkende Sekundärmetaboliten waren bisher in *Bacillus amyloliquefaciens* subsp. *plantarum* bisher nur die Polyketide Difficidin, Bacillaen und Makrolaktin, sowie das Dipeptid Bacilysin bekannt (siehe Erfindungsbeschreibung). Interessanterweise wurde in Mutantenstämmen, in denen die nicht-ribosomale Synthese von Polyketiden und Bacilysin eliminiert worden war, eine antibakterielle Aktivität gegenüber verschiedenen Gram-positiven Bakterien, u. a. Bacillus subtilis nachgewiesen (Tabelle 7). Zur Identifizierung des Genclusters, das für die Synthese dieser unbekannten, antibakteriellen Verbindung verantwortlich war, wurde eine Transposonmutagenese mit dem Mariner Transposon pMarA (leBreton et al. 2006) in dem Stamm CH5 (*sfp*), der nicht mehr zur nicht-ribosomalen Synthese antimikrobieller Sekundärmetaboliten befähigt war, durchgeführt. Mariner-Transposons wie pMarA können an beliebige Stellen des Chromosoms des bakteriellen Wirtes inserieren und dabei die Funktion des dort befindlichen Genes zerstören. Der das *mariner* Plasmid enthaltene Stamm AB10 wurde über Nacht bei 37°C und 210 rpm geschüttelt und anschließend mit steriler Saline 1:1000 verdünnt, davon 100 µl ausplattiert, 24 h bei 48°C inkubiert und die entstandenen Kolonien anschließend im Transposon-Mutanten-Screening getestet. Die Transposonmutanten wurden parallel jeweils auf eine Kanamycin-Platte und eine Diabrot-Platte (Zusammensetzung siehe unten) überführt. Die 10 x 10 cm große Platte mit Diabrot-Agar wurde zuvor mit 5 ml handwarmen LB Top Agar überschichtet, der 1:40 mit einer *Bacillus subtilis* HB0042 (OD ∼ 1) Kultur vermischt wurde. Die Kanamycin-Platten wurden ÜN bei 37°C inkubiert, die Diabrot-Platten bei Raumtemperatur. Am nächsten Tag wurden die auf den Diabrot-Platten gewachsenen Klone bezüglich eines Aktivitätsverlustes gegenüber *Bacillus subtilis* untersucht. Klone mit verminderter bzw. ohne Aktivität wurden von der parallel angefertigten Kanamycin-Platte selektiert und sequenziert. Von den Transposonmutanten wurde chromosomale DNA isoliert. 5 µg der DNA wurden mit Taql geschnitten und nach Aufreinigung mit dem Nucleospin Extract Kit mit dem Rapid Ligation Kit zirkularisiert (DNA Konzentration 5 ng/µl). Der Ligationsansatz wurde wiederum mit dem Nucleospin Extract Kit aufgereinigt und eine PCR mit einer Annealing-Temperatur von 60°C bzw. 50°C mit den Primern oIPCR1 und oIPCR2 durchgefóhrt. Die Transposon-Insertionsmutante WY01 wies keine antibakterielle Aktivitδt auf. Die Transpososon-Insertion wurde innerhalb des *acnA* Gens kartiert. *acnA* ist Teil eines Genclusters, das offensichtlich in die Synthese der unbekannten antibakteriellen Verbindung involviert ist (Abb. 1 a). AcnA kodiert den 112 AS Präkursor eines Peptids folgender Sequenz:

MMNLVKSNKKSFILFGAALAAATLVYALLLTGTELNVAAAHAFSANAE
LASTLGISTAAA
KKAIDIIDAASTIASIISLIGIVTGAGAISYAIVATAKTMIKKYGKKYAAAW

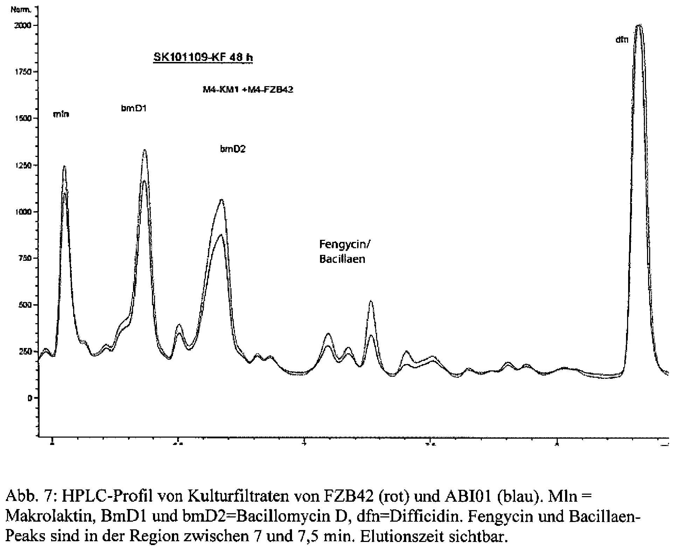

Die Sequenz der unterstrichenen 64 C-terminalen Aminosäuren weist Ähnlichkeit zu cyclischen Bacteriocinen, insbesondere dem Uberolysin (Wirawan et al. 2007) auf und stellt das reife, prozessierte Peptid dar. Die N-terminalen 48 Aminosäuren stellen wahrscheinlich das Leader Peptid dar. Durch den Vergleich mit dem Uberolysin-Präkursor und Bestimmung des vermutlichen Leader-Peptids ergibt sich ein Molekulargewicht von 6399,62 für das *acnA*-Produkt. Eine Verbindung mit diesem Molekulargewicht konnte allerdings nicht in dem methanolischen Extrakt des Ammoniumsulfat-gefällten Überstandes von ABI01 und anderen Vertretern von B. amyloliquefaciens plantarum, z.B. FZB42 und dessen Mutanten nachgewiesen werden. Allerdings wurde im MALDI-TOF Massenspektrum ein Peak mit der Masse von *m*/*z* 6382 Da [M+H]⁺ detektiert (Abb. 7a). Beim Vergleich der MALDI-TOF MS Spektren (90) des Methanolextraktes der *acnA* Mutante war dieser Peak nicht vorhanden; eine experimentelle Bestätigung dafür, dass es sich hier um das gesuchte, prozessierte Genprodukt von *acnA* handelt. Die um 18 Da (16 + 2) reduzierte Masse bestätigt den erfolgten Ringschluss, der wie bei anderen Repräsentanten der zyklischen Bacteriocine der Gruppe 1 zwischen der N-terminalen und der C-terminalen Aminosäure des prozessierten Peptids erfolgt:
Das prozessierte, reife Peptid besteht aus 64 Aminosäuren und weist eine "head to tail" Cyclisierung, d.h. eine kovalente Bindung zwischen L1 und W64, auf:
Sequenzen

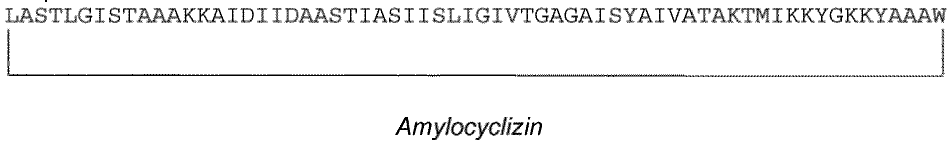
LASTLGISTAAAKKAIDIIDAASTIASIISLIGIVTGAGAISYAIVATAKTMIKKYGKKYAAAW


Amylocyclizin

Sequenzen mit Homologie zum acnA-F Gencluster von ABI01 finden sich auch in anderen Stämmen von *Bacillus amyloliquefaciens* (z.B. FZB42, DSM7) und *Bacillus subtilis* subsp. *spizizenii*, sowie *Bacillus coagulans* und *Paenibacillus larvae*. Zusammensetzung des Diabrot Mediums für die Produktion von Amylocyclizin 40 g Soja Pepton, 40 g Dextrin 10, 1.8 g KH₂PO₄, 4.5 g K₂HPO₄, 0.3 g MgSO₄ x 7 H₂O, und 0.2 ml KellyT Spurenelement-Lösung per L. KellyT Spurenelement-Lösung: 25 mg EDTA Dinatrium dihydrat, 0.5 g ZnSO₄ x 7 H₂O, 3.67 g CaCl₂ x 2 H₂O, 1.25 g MnCl₂ x 4 H₂O, 0.25 g CoCl₂ x 6 H₂O, 0.25 g Ammoniummolybdat, 2,5 g FeSO₄ x 7H₂O, 0,1 g CuSO₄ x 5 H₂O; eingestellt auf pH 6 mit NaOH, 500 ml H₂O.

Überstand: 50ml des Diabrot-Produktionsmediums in einem 500ml Rundkolben wurden mit 0,5 ml einer LB-Übernachtkultur beimpft und für 6,5 Stunden bis zu einer OD 8,5 unter Schütteln weiter inkubiert. Der Zellüberstand wurde durch zweifache Zentrifugation bei 14 000 U/min. (jeweils 10 min.) erhalten.

Bioassay. LB-Agar (20 ml) wurde mit 0.5 ml des Indikator Stammes (OD₆₀₀ ∼1.0) gemischt. 20 µl des Kultur-Überstandes wurden auf die Platte aufgetragen und für 16 h bei 22 °C inkubiert. Die Aktivität von Amylocyclicin A wurde als Klarzone beobachtet.

Ammonsulfatfällung des Überstandes: 50ml Überstand wurden mit 200ml H₂O dest. verdünnt und mit 80% Ammoniumsulfat Sättigung gefällt. Nach Zentrifugation (5min 14000 rpm) wurde das Sediment mit 250 µl Methanol aufgenommen und der methanolische Überstand nach erneuter Zentrifugation für die MALDI-TOF-MS Messung eingesetzt.

MALDI-TOF Massen Spektrometrie. MALDI-TOF Massen Spektren wurden mit einem Bruker Autoflex MALDI-TOF Gerät mit einem 337 nm Stickstoff Laser für Desorption und Ionisation aufgenommen. 2 µl Proben wurden mit gleichem Volumen Matrix-Lösung (gesättigte Lösung von α-Cyano-4-hydroxycinnamic Säure in 50% wässrigem Acetonitril mit 0.1% v/v Trifluoressigsäure) gemischt, aufgetragen, luftgetrocknet, und wie vorher beschrieben aufgetragen (Koumoutsi et al. 2006). Die Spektren wurden durch Detektion positiver Ionen im linearen Massenspektrum erhalten.

### Referenzen

1. Koumoutsi, A., X. H. Chen, A. Henne, H. Liesegang, G. Hitzeroth, P. Franke, J. Vater, and R. Borriss. 2004. Structural and functional characterization of gene clusters directing nonribosomal synthesis of bioactive cyclic lipopeptides in Bacillus amyloliquefaciens strain FZB42. J Bacteriol. 186:1084-96.
2. Le Breton, Y., N. P. Mohapatra, and W. G. Haldenwang. 2006. In vivo random mutagenesis of Bacillus subtilis by use of TnYLB-1, a mariner-based transposon. Appl Environ Microbiol 72:327-33.
3. Wirawan, R. E., K. M. Swanson, T. Kleffmann, R. W. Jack, and J. R. Tagg. 2007. Uberolysin: a novel cyclic bacteriocin produced by Streptococcus uberis. Microbiology. 153:1619-30.

### Beispiel 5: Bildung von Sekundärmetaboliten mit antimikrobieller Aktivität durch Bacillus amyloliquefaciens plantarum ABI01 (DSM 10-1092)

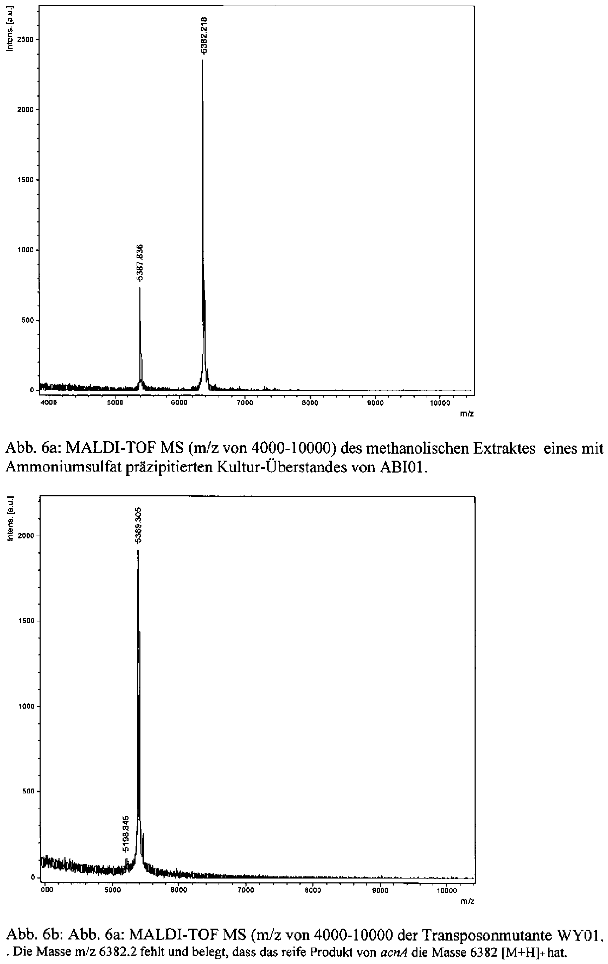

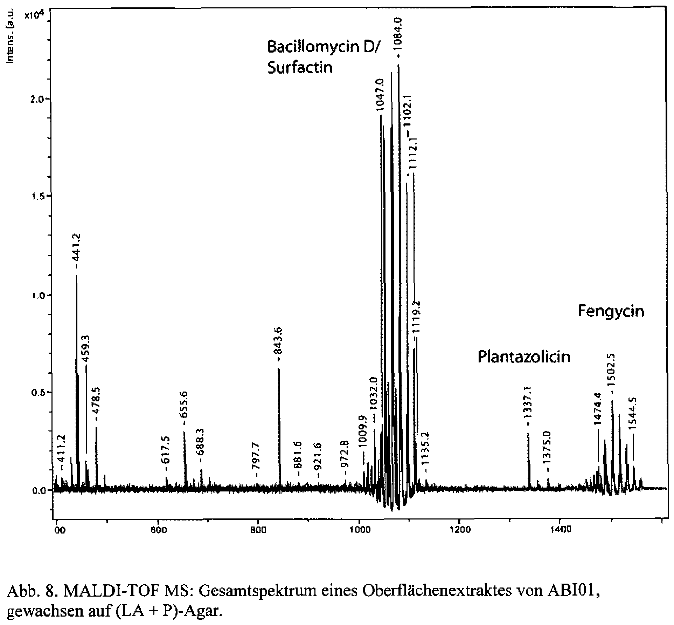

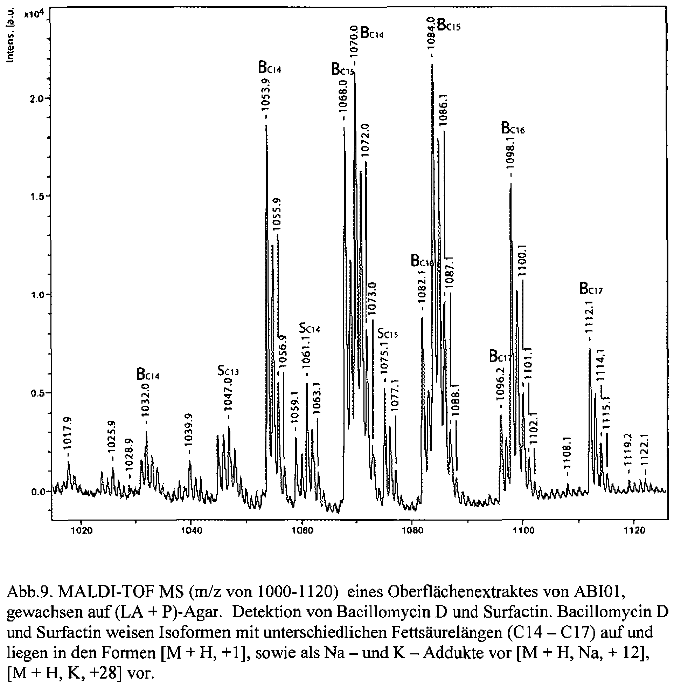

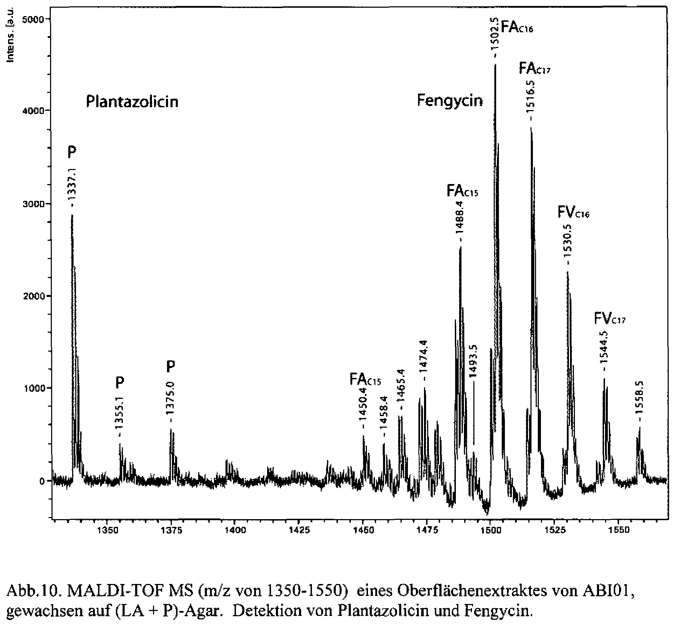

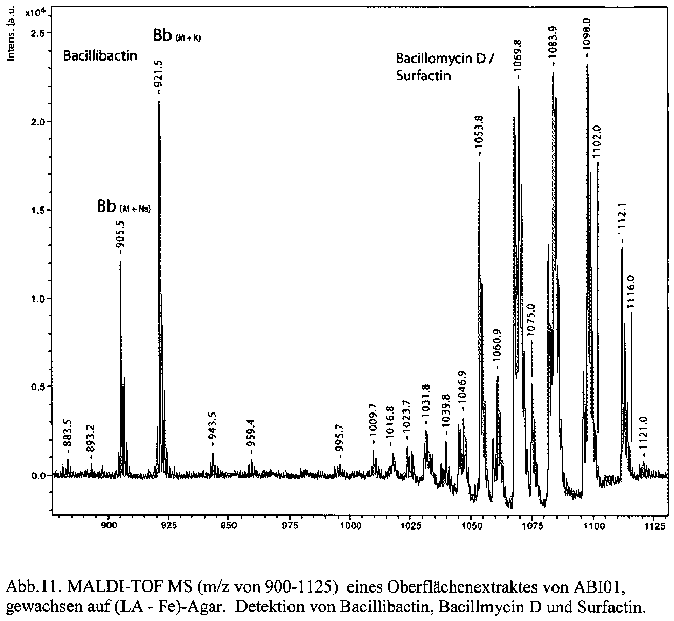

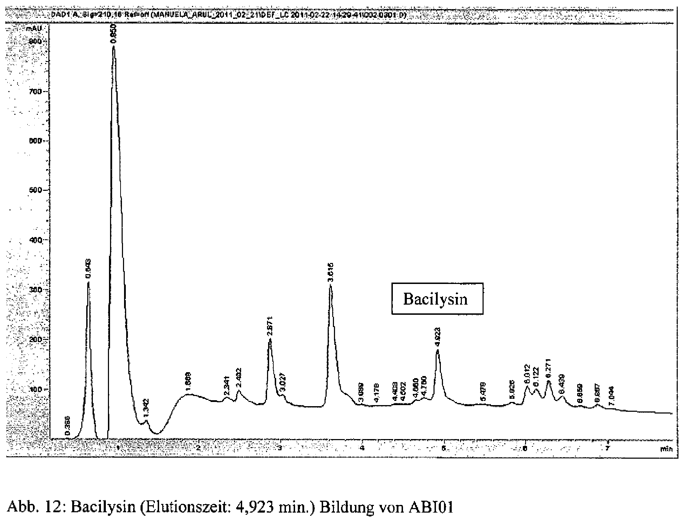

Durch ausgedehnte Untersuchungen mit *B.amyloliquefaciens plantarum* FZB42 und anderen Repräsentanten der BAP Gruppe ist bekannt, dass Vertreter dieser Gruppe bis zu neun verschiedene Sekundärmetaboliten mit antagonistischer Wirkung gegen die mikrobielle Mikroflora im Bereich der Pflanzenwurzel ribosomal bzw. nicht-ribosomal synthetisieren können (2-12). In der rechten Spalte der Tabelle 3 sind die Verbindungen im Fettdruck hervorgehoben, die durch Anwendung von MALDI-TOF MS ("matrix assisted laser desorption/ionisation mass spectrometry") und HPLC ("High pressure liquid chromatography") im Kulturfiltrat bzw. in Oberflächenextrakten von ABI01 nachgewiesen werden konnten. Die Ergebnisse sind in den Abbildungen 6 - 11 dokumentiert. Im Falle von **Bacillomycin D** wurden Isoformen mit unterschiedlicher Kettenlänge der mit dem Peptid verbundenen Fettsäurereste nachgewiesen (Abb. 7). Im Einzelnen handelt es sich um die Formen C14, C15, C16 und C17 (Abb 8). Es wird angenommen, dass die Länge der Fettsäuren einen Einfluss auf die antifungale Wirkung von Bacillomycin D ausübt. Bacillomcin D weist ebenso wie andere Mitglieder der Iturin-Familie (z.B. Iturin A) neben einer starken antifungalen Wirkung auch hämolytische Aktivität auf (Thimon et al. 1995). Im Bereich des Bacillomycin D Clusters sind auch Massen-Peaks des Lipopeptids **Surfactin** mit den Fettsäurekettenlängen C14 und C15 sichtbar (Abb. 8). Surfactin weist nur eine schwache antifungale Wirkung auf, ist aber für seine antivirale und oberflächenaktive Wirkung bekannt Peypoux et al. 1999). ABI01 bildet mit **Fengycin** eine zweites, antifungal wirkendes Lipopeptid, das ebenfalls durch MALDI-TOF MS von Oberflächenextrakten nachgewiesen werden konnte (Abb. 7). Durch das gleiche Gencluster von Peptid-Synthetasen werden in der Position 6 des Lipopeptids zwei unterschiedliche Aminosäuren, Alanin bzw. Valin eingebaut. Daneben existieren wie im Falle von Bacillomycin D und Surfactin Isoformen von Fengycin mit den FettsäureKettenlängen von C15, C16 und C17 (Abb. 9). Fengycin A enthält Ala in Position 6 während bei Fengycin B Ala durch Val ersetzt ist. Die antifungale Wirkung von Fengycin beruht wahrscheinlich auf der Hemmung von Phospholipase A₂ (Nishikori et al. 1986). Unter Eisenmangelbedingungen wird in ABI01 die Synthese des Eisen-Siderophors **Bacillibactin** induziert, das im Massenspektrum (Abb. 10) mit Peaks bei 905,5 [M + Na] und 921,5 [M + K] repräsentiert ist. Im Massenbereich um 1337, 1351 und 1371 war ein hoch-modifizierte Thiazol/Oxazol Mikrocin sichtbar, das als **Plantazolicin** beschrieben wurde, nachweisbar. Das konventionell (ribosomal) synthetisierte und prozessierte Peptid konnte kürzlich in *Bacillus amyloliquefaciens plantarum* nachgewiesen werden und weist antibakterielle Aktivität gegenüber nahe verwandten Gram-positiven Bakterien auf und wird durch ein 12-Gen Cluster kodiert (Scholz et al. 2011, Abb. 1a). **Bacilysin** [L-alanyl-[2,3-epoxycyclohexanone-4]-L-Alanin ist ein Dipeptid mit einem L-Alanin-Rest am N-Terminus und einer nichtproteinogenen Aminosäure, L-Anticapsin, am C-Terminus. Das Dipeptid supprimiert eine Vielzahl von Bakterien und die Hefe Candida albicans (Steinborn et al. 2005). Bacilysin wurde im Kulturfiltrat von ABI01, gewachsen in Glutamat-freiem Landy-Medium - durch HPLC nachgewiesen (Abb. 11). Die in ABI01 nicht-ribosomal synthetisierten Polyketide zeichnen sich durch eine hohe antibakterielle Aktivität aus. Sie wurden im Kulturfiltrat nach Wachstum von ABI01 in Landy-Medium durch HPLC nachgewiesen. **Bacillaen** besteht aus einer Vielzahl extrem-labiler, offenkettiger Isomere, darunter Bacillaen und Dihydrobacillaen als Hauptprodukte (Chen et al. 2009), die durch Oxidation schnell abbaubar sind. Repräsentanten dieser Stoffgruppe werden nach 7 - 7,5 min von der HPLC-Säule eluiert (Abb. 6). **Difficidin** und Oxydifficidin sind hochgradig ungesättigte makrozyklische Polyen Laktonphosphat Ester mit antibakterieller Breitband-Wirkung (Zimmermann et al. 1987). Sie werden nach 8,5 min. von der HPLC-Säule eluiert (Abb. 6). Dagegen erfolgt die Elution von **Makrolaktin** bereits nach 6 min. (Abb. 6). Makrolaktine enthalten drei getrennte Dien-Strukturelemente innerhalb eines Laktonringes, der aus 24 Elementen zusammengesetzt ist (Gustafson et al. 1989).

Probenvorbereitung: Die Probenvorbereitung für die HPLC der Lipopeptide und Polyketide erfolgte durch Behandlung mit dem Ionenaustauscher Amberlit (AD16) in folgender Weise:
1. 3 g AD16 werden in 20 ml Plastiksäulen eingefüllt und 3 x mit dest. Wasser gespült.
2. 5 ml Probe (Kulturüberstand der Bacillus-Kultur) werden auf die Säule gegeben und nachfolgend mit 3 x 10 ml dest. Wasser gespült.
3. Die an AD16 gebundenen Sekundärmetaboliten werden mit 3 x 2,5 ml Methanol eluiert.
4. Die Proben werden im Vakuum-Rotationsverdampfer bei 40°C bis zur Trocknung verdampft. Anschließend in 0,5 ml 90%igem Methanol unter Vortexen aufgenommen. Die Proben stehen jetzt für anschließende Bioautographie oder HPLC zur Verfügung.

### HPLC der Lipopeptide und Polyketide

Die Auftrennung der Proben durch HPLC ("high pressure liquid chromatography") wurde mit einem Agilent 1100 HPLC System (Agilent, Waldbronn, Deutschland) bei einer Flussrate von 1,5 ml/min. und einem Gradient von 0% Acetonitril + 0,1% Ameisensäure zu 100% Acetonitril + 0,1% Ameisensäure durchgeführt (Chen et al. 2006). 5 ml Probe wurden über eine MERCK LiChrolut RP-10 (500 mg) Säule nach Konditionierung der Säule mit 3 ml Methanol und 2 x 3 ml dest. Wasser aufgetragen. Dann wird mit 2 x 3 ml dest. Wasser und mit 2 x 3ml 50 %igem Methanol gewaschen. Die Säule wird 5 min. im Vakuum getrocknet, bevor die Eluierung mit 3 x 1,5 ml Methanol erfolgt.

### Massenspektroskopie der Lipopeptide und von Plantazolicin

Für den Nachweis von Lipopeptiden von ganzen Zellen, *B. amyloliquefaciens plantarum* wurde auf Agar-Platten mit Landy-Medium (Landy et al. 1948) drei Tage bei 30°C angezogen. Zur Erstellung der Oberflächenextrakte für die Massenspektroskopie (MALDI TOF MS, Leenders et al. 1999) wurde Zellmaterial von den Agarplatten entnommen, in 0,5 ml Eppendorf-Tubes überführt und mit 100 mikroLiter Matrix-Medium (50% Acetonitril/ 0,1%Trifluor-Essigsäure) extrahiert.

### Referenzen

Chen, X.-H et al. 2006. Structural and functional characterization of three polyketide synthase gene clusters in Bacillus amyloliquefaciens FZB42. J. Bacteriol. 188:4024-4036
Chen, X-H., Koumoutsi, A., Scholz, R., Borriss, R. 2009. More than anticipated-Production of antibiotics and other secondary metabolites by Bacillus amyloliquefaciens FZB42. J. Mol. Microbiol. Biotechnol 16:14-24
Gustafson, K., M. Roman, W. finical 1989. The macrolactins, a novel class of antiviral and cytotoxic macrolides from a deep-sea marine bacterium. J. Am. Chem. Soc. 111:7519-7524
Landy, M. et al. 1948. Bacillomycin, an antibiotic from Bacillus subtilis active against pathogenic fungi. Proc. Soc. Exp. Biol. Med. 67:539-541
Leenders, F., T. H. Stein, B. Kablitz, P. Franke, J. Vater. 1999. Rapid typing of Bacillus subtilis strains by their secondary metabolites using matrix assisted laser desorption/ionization mass spectrometry of intact cells. Rapid Commun. Mass Spectrom. 13: 943-949
Nishikori T., H. Naganawa, Y. Muraoka, T. Aoyagi, H. Umezawa. 1986. Plipastatins: new inhibitors of phospholipase A2, produced by Bacillus cereus BMG302-fF67. III. Structural elucidation of plipastatins. J. Antibiot. 39:755-761
Peypoux, F., J. M. Bonmatin, J. Wallach 1999. Recent trends in the biochemistry of surfactin. Appl. Microbiol. Biotechnol. 51:553-563
Scholz et al. 2011. Plantazolicin, a novel microcin B17/streptolysin S-like natural product from Bacillus amyloliquefaciens FZB42. J. Bacteriol. 193: 215-224
Steinborn, G., Hajirezaei, M-R, Hofemeister, J. 2005. Bac genes for recombinant bacilysin and anticapsin production in Bacillus host strains. Arch Microbiol 183:71-79 Thimon, L., Peypoux, F., Wallach, J., Michel, G. 1995. Effect of the lipopeptide antibiotic, iturin A on morphology and membrane structure of yeast cells. FEMS Microbiol. Lett. 128: 101-106
Zimmerman, S.B., C. D. Schwartz, R. L. Monaghan, B. A. Belak, et al. 1987. Difficidin and oxydifficidin: novel broad spectrum antibacterial antibiotics produceed by Bacillus subtilis. 1. Production, taxonomy and antibacterial activity. J. Antibiot. 40:1677-1681

### Beispiel 6 : Herstellung einer Sporensuspension

Im Detail wird *Bacillus amyloliquefaciens plantarum* ABI01 auf ein herkömmliches Medium geimpft, das eine geeignete Kohlenstoffquelle, Stickstoffquelle und anorganische Verbindungen aufweist, und bei bestimmter Temperatur und pH unter aeroben Bedingungen kultiviert. Beispiele der Kohlenstoffquelle beinhalten Glucose, Melasse, Lactose, Sucrose, Maltose, Dextrin, Stärke, Mannitol, Sorbitol und Glycerin. Bevorzugt werden Glucose und Stärke verwendet. Beispiele der Stickstoffquelle beinhalten eine anorganische Quelle wie Ammoniak, Ammoniumchlorid und Ammoniumsulfat und eine organische Quelle wie Magermilchpulver, Casein, Pepton, NZ-Amin, Rindfleischextrakt, Hefeextrakt, Maisquellwasser (corn steep liquor), Caseinhydrolysat, Fisch oder Fischmehl und entfettete Sojabohne oder Sojamehl. Bevorzugt werden Hefeextrakt oder Maislauge gewählt. Beispiele der anorganischen Verbindungen beinhalten Kaliummonohydrogenphosphat, Kaliumdihydrogenphosphat, Magnesiumsulfat, Eisensulfat, Mangansulfat und Calciumcarbonat. Für die Kultur kann Schüttelkultur oder Rührkultur mit Belüftung verwendet werden. Die Kulturtemperatur liegt im Bereich von 25 bis 45 °C, und bevorzugt 28° bis 35 °C. Bei der Kultur wird der pH bevorzugt auf einen ziemlich neutralen Wert eingestellt. Die Kulturdauer beträgt 1 bis 2 Tage bis mehr als 90% der vegetativen Zellen zu hitzeresistenten Endosporen umgewandelt sind. Anschließend werden die Sporen im Durchlaufseparator isoliert und mit ca. 10% des gesamten Kulturvolumens aufgenommen. Der Zelltiter des Sporenkonzentrats beträgt mehr als 10¹⁰ cfu/ml.

Gemäß einer Ausführungsform der vorliegenden Erfindung wird der *Bacillus amyloliquefaciens plantarum* ABI01 auf ein Vorkulturmedium geimpft und bei einer Belüftungsströmungsrate von 1 vvm, 37 °C und 800 Upm 20 Stunden lang kultiviert. Die Vorkultur wird auf ein Fermentationsmedium überimpft und einer Schüttelkultur bei einer Belüftungsströmungsrate von 1 vvm, 40 °C, 800 Upm und pH 7,0 über 60 bis 70 Stunden unterzogen. Bei der Schüttelkultur wird die Fermentierungskultur mit einem Glucosezusatzmedium versorgt, das die restliche Glucose in der Kultur auf einem Wert von 0,5 bis 1 % hält, bis der Gesamtgehalt an Glucose in der Fermentierungskultur 20 % erreicht.

### Beispiel 7: Anwendung einer Sporensuspension von ABI01 bei mit Rhizoctonia solani infizierten Kartoffeln

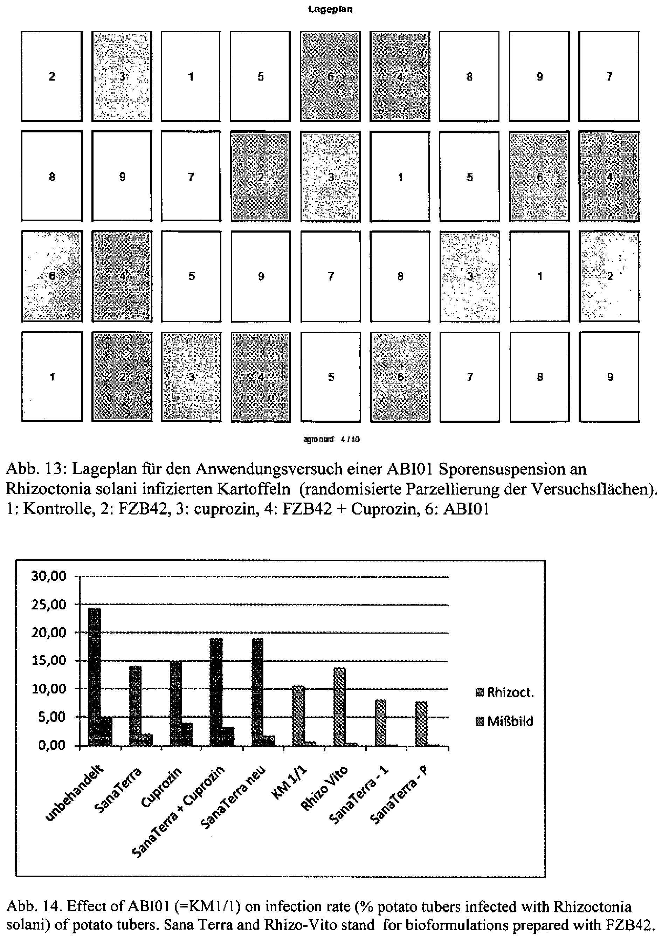

Die Versuche wurden mit Kartoffelpflanzen, die durch Rhizoctonia solani infiziert waren, in Groß-Lüsewitz (Mecklenburg-Vorpommern) durch Agro-Nord (Dr. Kürtzinger) 2010 (Mai bis August) durchgeführt. Die Untersuchung erfolgte in einem randomisiert parzellierten Versuchsfeld (Abb. 13). Unmittelbar vor der Behandlung (30.04. 2010) ergab eine Bonitur der Kartoffelknollen, dass 36% frei von Befall waren, 30% eine geringe Befallsdichte (Klassen 1-5) aufwiesen, 14% mittel (Klassen 6-10), 13 % stark (Klassen 11-15) und 14% sehr stark (>Klasse 15) mit Rhizoctonia Sklerotien infiziert waren. Zum Einsatz kamen die folgenden Varianten: 1 (Kontrolle ohne Applikation), 2 (FZB42 Sporensuspension "Sana Terra", 0,5 L/ha), 3 (Chemisches Fungizid Cuprozin, 0,4 L/ha), 4 ("Sana Terra 0,5 L/ha + Cuprozin 0,4 L/ha), 6 (ABI01 Sporensuspension 0,5 L/ha). Die Konzentration der Sporensuspensionen von ABI01 und FZB42 betrug 2,5 x 10¹⁰ Sporen/g. Die Behandlung der Knollen erfolgt bei der Abgabe vom Doppelbecherband in die Pflanzenfurche. Bei einer Pflanzgutmenge von z.B. 25 dt/ha wurden 0,5 L der Sporensuspension /ha in ca. 80 l Wasser/ha verdünnt und mittels Vollkegeldüsen auf die in die Furche fallenden Kartoffeln gesprüht. Es wurden 100 Knollen/Parzelle gelegt. Die 2. Bonitur wurde am 30. 06. 2010 (2 Monate nach Pflanzung) durchgeführt und zeigte, dass bei den Varianten 2, 3, 4, und 6 mindestens 100% im Vergleich zur Kontrolle (1) aufgelaufen waren. Das entspricht einer Anzahl von ca. 75 Knollen/Parzelle. Die 4. Bonitur wurde am 13. 10. 2010 (Ernte, ca. 5,5 Monate nach dem Legen der Kartoffeln) durchgeführt.

Die in der Tabelle 8 dokumentierten Ergebnisse belegen eine etwa gleichartige fungizide Wirkung des chemischen Fungizids Cuprozin (Variante 3) und des biologischen Pflanzenschutzmittels FZB42 (Variante 2), die zu einer Reduktion der durch *Rhizoctonia solani* verursachten Wurzeltöterkrankheit ("black scurf") von 24,33% (Kontrolle) auf 15,00 % (Variante 3) bzw. 14,08% (Variante 2) führten. Die beste Wirkung wurde mit der Sporensuspension von ABI01 (Variante 4) erzielt. Hier konnte eine Reduktion des Befalls durch *Rhizoctonia solani* auf 10,58% aller untersuchten Knollen erreicht werden.

### Beispiel 8: Anwendung einer Sporensuspension von B. amyloliquefaciens subsp. plantarum bei Tabakpflanzen

Das Experiment wurde in der Zeit vom 15. Mai bis zum 26. September 2010 auf dem Feld von Chen Dibin bei two village Shunzhou town Yongsheng county Lijiang city durchgeführt. Höhe war 2207m, N26.61555 nördlicher Breite und E100.5561 Länge. Der Boden wies eine mittlere Fruchtbarkeit auf und bestand aus lehmigen Ton (25,6% Ton, 48,2 % Sand und 26,2% Schluff). Weitere Bodenparameter: Pflugtiefe 21 cm, pH 5,4, organische Bestandteile: 40.60g/kg, verfügbarer Stickstoff (N) 152,10 mg/kg, verfügbarer Phosphor (P): 12,23 mg/kg, Kalium (K) 485.41mg/kg. Vorhergehende Bepflanzung: Brache, verschiedene Tabakvarietäten. Pflanzgröße: 120 cm x 50 cm (1100 Tabakpflanzen per Mu). Die Bepflanzung wurde am 19. Mai 2010 vorgenommen.

Es wurden 5 Varianten mit jeweils drei Wiederholungen auf insgesamt 15 Parzellen durchgeführt. Jede Versuchsparzelle (8,5 m x 3,6 m = 30,6 m²) wurde mit 60 Pflanzen bepflanzt. Die Gesamtfläche des Versuchsfeldes war 1,2 Mu. Der Einsatz von FZB42 Sporensuspension (2,5 x 1010 Sporen/ml) in einer Konzentration von 33 mL /Mu (0,5 L/ha) erfolgte durch Angießen während der Bepflanzung der Versuchsflächen (Variante 4), bzw. Besprühen **nach** dem Anwachsen der Pflanzen (Variante 5). Vor Anwendung wurden die Sporensuspensionen mit Leitungswasser 1:10000 bzw. 1: 2000 verdünnt. Anschließend wurde ausreichend mit Wasser gegossen. Die Kontrollen wurden mit Wasser (Variante 1) bzw. mit chinesischem Biodünger (40-60 g/Mu) zur Pflanzungszeit (Variante 2) bzw. nach dem Anwachsen der Pflanze als "topdressing" (60-100 g/Mu, Variante 3) durchgeführt. Drei Bonituren, die nach 46, 65 und 81 Tagen nach Bepflanzung durchgeführt wurden, ergaben, dass durch die Anwendung von FZB42 der Krankheitsbefall der Tabakpflanzen deutlich vermindert werden konnte (nicht nur gegenüber der Kontrolle, sondern auch gegenüber den Varianten 2 und 3, die mit einem chinesischen Biodünger behandelt worden waren. Als besonders vorteilhaft erwies sich die Variante 4, bei der die Behandlung mit der Sporensuspension unmittelbar bei der Pflanzung erfolgte (Tabelle 9). Im Einzelnen kam es zur statistisch signifikanten Unterdrückung der folgenden Krankheiten, die bei allen drei durchgeführten Bonituren gleichermaßen festgestellt werden konnte:
1. Tabakmosaikvirus (TMV): 1. Bonitur: 3,27 % (FZB42) /27,78 (Kontrolle); 2. Bonitur: 9,80 % (FZB42)/ 42,48% (Kontrolle); 3. Bonitur: 16,34 % (FZB42)/ 84,97 (Kontrolle)
2. Bakterielle Tabakwelke (*Ralstonia solanaceum*): 1. Bonitur: 0,00 % (FZB42) /9,80 (Kontrolle); 2. Bonitur: 3,27 % (FZB42)/ 19,61% (Kontrolle); 3. Bonitur: 9,8 % (FZB42)/ 29,41 (Kontrolle)
3. Braunfleckenkrankheit (*Alternaria alternata*): 1. Bonitur: 0,00 % (FZB42) /23,53 % (Kontrolle); 2. Bonitur: 15,69 % (FZB42)/ 62,75 % (Kontrolle); 3. Bonitur: 31,37 % (FZB42)/ 105,88 (Kontrolle)
4. Echter Mehltau (*Erisyphe cichoracearum*) : 1. Bonitur: 6,54 % (FZB42) /43,14 % (Kontrolle); 2. Bonitur: 27,45 % (FZB42)/ 94,12 % (Kontrolle); 3. Bonitur: 52,94 % (FZB42)/ 117,65 % (Kontrolle)
5. "Climatic spot" (Fleckenkrankheit durch abiotischer Stress): 1. Bonitur: 0,00 % (FZB42) /7,84 % (Kontrolle); 2. Bonitur: 27,45 % (FZB42)/ 43,14 % (Kontrolle); 3. Bonitur: 52,94 % (FZB42)/ 117,65 % (Kontrolle)

Neben verbesserter Krankheitsresistenz, einschließlich viraler und bakterieller Erkrankungen, zeigten die Ergebnisse, dass der Zusatz von Stämmen der Subspecies *B. amyloliquefaciens plantarum* zu verbessertem Wachstum und erhöhten Erträgen bei den Tabakpflanzen führt. So konnte bei Anwendung von FZB42 (Variante 4) eine Ertragssteigerung von 10,88% gegenüber der Kontrolle erzielt werden. Insgesamt wurde eine Wertsteigerung der geernteten Tabakblätter per Mu von 15,68% erzielt.

### Eigene Patentanmeldungen:

1. Europäische Patentanmeldung EP 0 8015 973.4
   Antibakterielles Mittel zur Behandlung von Feuerbrand bei Obstgehölzen und anderen bakteriell verursachten Pflanzenkrankheiten
   Anmelder: ABITEP GmbH
2. Deutsche Patentanmeldung DE 11 2004 001 054.3
   "Sequenz für die BacillomycinD Synthese in Bacillus amyloliquefacienFZB42 Anmelder Prof. Dr. Rainer Borriss
3. Deutsche Patentanmeldung DE 10 20040 273 35.9
   "Sequenzen für die Synthese antibakteriell wirkender Polyketide" Anmelder Prof. Dr. Rainer Borriss

### In der Patentschrift angeführte Literatur:

*1*. Borriss R, Chen X, Rueckert C, Blom J, Becker A, Baumgarth B, Fan B, Pukall R, Schumann P, Spröer C, Junge H, Vater J, Pühler A, Klenk HP. Relationship of Bacillus amyloliquefaciens clades associated with strains DSM7T and FZB42: a proposal for Bacillus amyloliquefaciens subsp. amyloliquefaciens subsp. nov. and Bacillus amyloliquefaciens subsp. plantarum subsp. nov. based on their discriminating complete genome sequences. Int J Syst Evol Microbiol. 2010 Sep 3*. [Epub ahead of print]*
2. Rückert C, Blom J, Chen X, Reva O, Borriss R. J Biotechnol. 2011 Genome sequence of B. amyloliquefaciens type strain DSM7 (T) reveals differences to plant-associated B. amyloliquefaciens FZB42. J Biotechnol. Jan 22. [Epub ahead of print]
3. Fan B, Chen XH, Budiharjo A, Bleiss W, Vater J, Borriss R. Efficient colonization of plant roots by the plant growth promoting bacterium Bacillus amyloliquefaciens FZB42, engineered to express green fluorescent protein J Biotechnol. 2011 Jan 13. [Epub ahead of print]
4. Scholz R, Molohon KJ, Nachtigall J, Vater J, Markley AL, Süssmuth RD, Mitchell DA, Borriss R. Plantazolicin, a novel microcin B17/streptolysin S-like natural product from Bacillus amyloliquefaciens FZB42. J Bacteriol. 2011 Jan;193(1):215-24
5. Chen XH, Scholz R, Borriss M, Junge H, Mögel G, Kunz S, Borriss R. Difficidin and bacilysin produced by plant-associated Bacillus amyloliquefaciens are efficient in controlling fire blight disease. J Biotechnol. 2009 Mar 10;140(1-2):38-44.
6. Chen XH, Koumoutsi A, Scholz R, Schneider K, Vater J, Süssmuth R, Piel J, Borriss R. Genome analysis of Bacillus amyloliquefaciens FZB42 reveals its potential for biocontrol of plant pathogens J Biotechnol. 2009 Mar 10;140(1-2):27-37
7. Chen XH, Koumoutsi A, Scholz R, Borriss R. More than anticipated - production of antibiotics and other secondary metabolites by Bacillus amyloliquefaciens FZB42. J Mol Microbiol Biotechnol. 2009;16(1-2):14-24.
8. Chen XH, Koumoutsi A, Scholz R, Eisenreich A, Schneider K, Heinemeyer I, Morgenstern B, Voss B, Hess WR, Reva O, Junge H, Voigt B, Jungblut PR, Vater J, Süssmuth R, Liesegang H, Strittmatter A, Gottschalk G, Borriss R. Comparative analysis of the complete genome sequence of the plant growth-promoting bacterium Bacillus amyloliquefaciens FZB42. Nat Biotechnol. 2007 Sep;25(9):1007-14.
9. Idris EE, Iglesias DJ, Talon M, Borriss R. Tryptophan-dependent production of indole-3-acetic acid (IAA) affects level of plant growth promotion by Bacillus amyloliquefaciens FZB42. Mol Plant Microbe Interact. 2007 Jun;20(6):619-26
10. Chen XH, Vater J, Piel J, Franke P, Scholz R, Schneider K, Koumoutsi A, Hitzeroth G, Grammel N, Strittmatter AW, Gottschalk G, Süssmuth RD, Borriss R. Structural and functional characterization of three polyketide synthase gene clusters in Bacillus amyloliquefaciens FZB 42. J Bacteriol. 2006 Jun;188(11):4024-36.
11. Koumoutsi A, Chen XH, Henne A, Liesegang H, Hitzeroth G, Franke P, Vater J, Borriss R. Structural and functional characterization of gene clusters directing nonribosomal synthesis of bioactive cyclic lipopeptides in Bacillus amyloliquefaciens strain FZB42. J Bacteriol. 2004 Feb;186(4):1084-96.
12. Idriss EE, Makarewicz O, Farouk A, Rosner K, Greiner R, Bochow H, Richter T, Borriss R. Extracellular phytase activity of Bacillus amyloliquefaciens FZB45 contributes to its plant-growth-promoting effect. Microbiology. 2002 Jul;148(Pt 7):2097-109.
13. Sambrook, J., Fritsch, E.F., Maniatis, T. Molecular cloning a laboratory manual (2th ed.) CSHLP, New York 1989

### Tabellen

**Tabelle 1: Gencluster von ABI01 involviert in die Synthese antimikrobieller Verbindungen**

| Genom ABI01 (bp von bis) | | Gene | Größe (kb) | Produkt¹ |
|---|---|---|---|---|
| Nicht-ribosomale Synthese (Lipopeptide, Peptide) | | | | |
| 342621 | 368806 | *srfAA srfAB (comS) srfAC srfAD* | 26,186 | **Surfactin** |
| 1969272 | 1931332 | *fenA, fenB, fenC, fend, fenE* | 37,670 | **Fengycin** |
| 1908426 | 1871134 | *bmyD, bmyA, bmyB, bmyC* | 37,293 | **Bacillomycin D** |
| 2886201 | 2868684 | *nrsA nrsB nrsC nrsD nrsE nrsF* | 14,374 | Putatives Peptid |
| 3031323 | 3019354 | *dhbA dhbC dhbE dhbB dhbF mbtH* | 11,970 | **Bacillibactin** |
| 3598101 | 3592212 | *bacA bacB bacC bacD bacE bacF ywfG* | 5,890 | **Bacilysin** |

| Nicht-ribosomale Polyketidsynthese | | | | |
|---|---|---|---|---|
| 1391843 | 1445096 | *mlnA mlnB mlnC mlnD mlnE mlnF mlnG mlnH mlnI* | 53,254 | **Makrolactin** |
| 1700352 | 1772793 | *baeB baeC baeD baeE acpK baeG baeH baeI baeJ baeL baeM baeN baeR baeS* | 72,442 | **Bacillaen** |
| 2346539 | 2277016 | *dfnA dfnY dfnX dfnB dfnC dfnD dfnE dfnF dfnG dfnH dfnI dfnJ dfnK dfnL dfnM* | 69,524 | **Difficidin** |

| Ribosomale Synthese antimikrobieller Peptide | | | | |
|---|---|---|---|---|
| 726474 | 736429 | *pznF pznK pznG pznH pznI pznA pznJ pznC pznD pzn B pznE pznL* | 9,956 | **Plantazolicin** |
| 3047281 | 3042815 | *acnF acnA acnB acnC acnD acnE* | 4,467 | **Amylocyclicin** |

| | | | | |
|---|---|---|---|---|
| ¹ Die fettgedruckten Verbindungen wurden durch HPLC bzw. MALDI TOF MS direkt im Kulturfiltrat oder zellgebunden bei ABI01 nachgewiesen. | | | | |

**Tabelle 2: Beispiel für die Charakterisierung von Isolaten aus Bodenproben - entnommen fünf Monate nach Anwendung von FZB42 zu Antirhinum major Kulturen, Chengong County, Kunming. Für die PCR wurde eine Hybrisierungstemperatur von 55°C benutzt.**

| Strain | Lactose MM | Cellulose | RM (785 bp) | Nrs (839 bp) | Pzn (821 bp) | Morphologie (Nähragar) |
|---|---|---|---|---|---|---|
| FZB42 | + | + | 785 bp | 839 bp | 821 bp | Rauh, flach, dendritisch, weiss bis durchscheinend |
| KM 1-1 | + | + | 785 bp | 839 bp | 821 bp | wie FZB42 |
| KM 1-2 | + | + | 785 bp | 839 bp | 821 bp | wie FZB42 |
| KM 1-3 | + | + | 785 bp | 839 bp | 821 bp | wie FZB42 |
| KM 2A | + | + | 785 bp | 839 bp | 821 bp | wie FZB42 |
| KM 2B | + | + | 785 bp | 839 bp | 821 bp | wie FZB42 |
| KM3 | + | + | 785 bp | 839 bp | 821 bp | wie FZB42 |
| KM 4-1 | + | + | 785 bp | 839 bp | 821 bp | wie FZB42 |
| KM 4-2 | + | + | 785 bp | 839 bp | 821 bp | wie FZB42 |
| KM 5-1 | + | + | 785 bp | 839 bp | 821 bp | wie FZB42 |
| KM 5-2 | + | + | 785 bp | 839 bp | 821 bp | wie FZB42 |
| KM 6-1 | + | + | 785 bp | 839 bp | 821 bp | wie FZB42 |
| KM 6-2 | + | + | 785 bp | 839 bp | 821 bp | wie FZB42 |
| DSM7^{T} | + | - | - | - | - | rauh, weiß |
| *B. subtilis* DSM10^{T} | - | + | - | - | - | kremig |

**Tabelle 3: Eigenschaften von B. amyloliquefaciens plantarum ABI01 (DSM 10-1092)**

| | |
|---|---|
| Eigenschaften | Ergebnis |
| Morphologie | Stäbchen |
| Durchmesser | 0,7-0,8 µm |
| Länge | 2,5 - 3,0 µm |
| Endo-Sporen | oval, ellipsoid |

| Sporangium nicht geschwollen | |
|---|---|
| Aminopeptidase | - |
| KOH Test | - |
| Oxidase | + |
| Katalase | + |
| Wachstum anaerob | - |
| Voges Proskauer Reaktion (VP) | + |
| pH in VP | 6,9 |
| Wachstum bei 50°C | + |
| Wachstum bei 55°C | - |
| Wachstum bei pH 5,7 | + |
| Wachstum mit 2% NaCl | + |
| Wachstum mit 5% NaCl | + |
| Wachstum mit 7% NaCl | + |
| Wachstum mit 10% NaCl | schwach |
| Wachstum in Lysozym-Nährlösung | + |
| Säurebildung von D-Glucose | + |
| Säurebildung von L-Arabinose | + |
| Säurebildung von D-Xylose | + |
| Säurebildung von D-Mannit | + |
| Säurebildung von D-Fruktose | + |
| Gas von Glukose | - |
| Hydrolyse von Stärke (Amylase) | + |
| Hydrolyse von Gelatine (Protease) | + |
| Hydrolyse von Casein (Protease) | + |
| Hydrolyse von Tween 80 (Lipase) | + |
| Hydrolyse von HE Cellulose (Cellulase, endo 1,4 ß-glucanase) | + |
| Lecithinase | + |
| Hydrolyse von Äsculin | + |
| Nutzung von Zitrat | + |
| Nutzung von Propionat | - |
| NO₂ von NO₃ (Nitratreduktase) | + |
| Indolreaktion | - |
| Phenylalanin Desaminase | - |
| Arginin Dihydrolase | - |

**Tabelle 4: Vergleichende Charakterisierung der Genome von FZB42 und ABI01**

| | FZB42 | ABI01 |
|---|---|---|
| Genomgröße (bp) | 3 918 591 | 3 913 505 |
| G + C Gehalt (mol %) | 46,49 | 46,47 |
| Protein kodierende Sequenzen (CDS) | 3 660 | 3 689 |
| Anzahl gemeinsamer CDS | 3590 | |
| Anzahl unikaler Sequenzen | 70 | 34 |
| Ribosomale RNA Operons | 10 | 10 |
| Anzahl von t-RNAs | 101 | 93 |

**Tabelle 5: Unikale Gene in ABI01. Keine Annotation in FZB42**

| Akzessions-Nummer | Start | Stop | Gen, Genprodukt |
|---|---|---|---|
| B._amyloliquefaciens_ABI01_320 | 217501 | 217004 | yxzK, UPF0299 membrane protein YE2790 |
| B._amyloliquefaciens_ABI01_375 | 263645 | 263439 | hypothetical protein predicted by Glimmer/Critica |
| B._amyloliquefaciens_ABI01_656 | 540780 | 540079 | hypothetical protein predicted by Glimmer/Critica |
| B._amyloliquefaciens_ABI01_710 | 587120 | 586935 | ycsA K07246 tartrate dehydrogenase/ decarboxylase / D-malate dehydrogenase 1.1.1.83 |
| B._amyloliquefaciens_ABI01_796 | 673777 | 673544 | yezF hypothetical protein |
| B._amyloliquefaciens_ABI01_827 | 713259 | 713555 | Nuclear pore complex protein Nup214 |
| B ._amyloliquefaciens_ABI0 1_8472 | 729981 | 729855 | pznA, plantathiazolicin precursor peptide |
| B._amyloliquefaciens_ABI01_1124 | 990112 | 989873 | yhzD hypothetical protein |
| B._amyloliquefaciens_ABI01_1141 | 1001924 | 1002103 | yhzF hypothetical protein |
| B._amyloliquefaciens_ABI01_1324 | 1164775 | 1164485 | yxxE Uncharacterized protein YxxE |
| B._amyloliquefaciens_ABI01_1359 | 1201030 | 1200845 | yoyH hypothetical protein |
| B._amyloliquefaciens_ABI01_1384 | 1220947 | 1221111 | xkdB Phage-like element PBSX protein xkdB |
| B._amyloliquefaciens_ABI01_1889 | 1776428 | 1776661 | conserved hypothetical protein |
| B._amyloliquefaciens_ABI01_1894 | 1776651 | 1778974 | hypothetical protein predicted by Glimmer/Critica |
| B._amyloliquefaciens_ABI01_1990 | 1861014 | 1861328 | hypothetical protein predicted by Glimmer/Critica |
| B._amyloliquefaciens_ABI01_2125 | 2051554 | 2051754 | yhjC hypothetical protein |
| B._amyloliquefaciens_ABI01_2167 | 2080107 | 2080355 | hypothetical protein predicted by Glimmer/Critica |
| B._amyloliquefaciens_ABI01_2224 | 2127845 | 2128120 | yoqM SPBc2 prophage-derived uncharacterized protein yoqM |
| B._amyloliquefaciens_ABI01_2307 | 2199107 | 2199238 | ypzI hypothetical protein |
| B._amyloliquefaciens_ABI01_2610 | 2507145 | 2507285 | yqzM hypothetical protein |
| B._amyloliquefaciens_ABI01_2685 | 2576412 | 2576281 | yrzQ hypothetical protein |
| B._amyloliquefaciens_ABI01_2713 | 2606042 | 2605812 | yrzS hypothetical protein |
| B._amyloliquefaciens_ABI01_2776 | 2662583 | 2662933 | Transcription elongation factor SPT5 |
| B._amyloliquefaciens_ABI01_2880 | 2759906 | 2760097 | ytzJ hypothetical protein |
| B._amyloliquefaciens_ABI01_3019 | 2889888 | 2890082 | hypothetical protein predicted by Glimmer/Critica |
| B._amyloliquefaciens_ABI01_3036 | 2906182 | 2906331 | hypothetical protein predicted by Glimmer/Critica |
| B._amyloliquefaciens_ABI01_3091 | 2958339 | 2958076 | yuzI hypothetical protein |
| B._amyloliquefaciens_ABI01_3092 | 2958472 | 2958726 | mstX hypothetical protein |
| B._amyloliquefaciens_ABI01_3229 | 3088401 | 3088150 | hypothetical protein predicted by Glimmer/Critica |
| B._amyloliquefaciens_ABI01_3241 | 3098142 | 3097990 | hypothetical protein predicted by Glimmer/Critica |
| B._amyloliquefaciens_ABI01_3606 | 3446061 | 3445666 | hypothetical protein predicted by Glimmer/Critica |
| B._amyloliquefaciens_ABI01_3634 | 3464594 | 3464448 | ywzD hypothetical protein |
| B._amyloliquefaciens_ABI01_3679 | 3501445 | 3501281 | hypothetical protein predicted by Glimmer/Critica |
| B._amyloliquefaciens_ABI01_3978 | 3760704 | 3760537 | yxeC Uncharacterized protein yxeC |
| B._amyloliquefaciens_ABI01_4039 | 3824161 | 3823991 | yyzF hypothetical protein |

**Tabelle 6: Statistisch gesicherte Sequenzunterschiede von Abi01 und FZB42 innerhalb von CDS**

| **Abi01** | **start** | **stop** | **FZB42** | **Austausch (Nukleotid und AS)** | **Identität** | |
|---|---|---|---|---|---|---|
| ABI01 _324 | 219939 | 220652 | RBAM02470¹ | - → T (220363), STOP(143) | | 142/143 |
| ABI01_344 | 239330 | 237156 | RBAM02650 | T→C (23876), S→G (190) | 2174/2175 | 723/724 |
| ABI01_719 | 591951 | 592550 | RBAM06150 | A→G (592339) G→A (592356), T→A (592366), G→T (592511), K→R (137), Q→K (140), G→W (180) | 594/599 | 196/199 |
| ABI01_720 | 593762 | 592602 | RBAM06160¹ | G→T (592578), C→G (592584), W→L (381), T→R (383) | 1159/1161 | 384/386 |
| ABI01_881 | 764576 | 765364 | RBAM07750 | 3xNt, 1xdel (765254), 2xins 765263) | 720/726 | 156/156 |
| ABI01_926 | 808660 | 807827 | RBAM08170 | G→C (807887), L→V (265) | 833/834 | 276/277 |
| ABI01 1002 | 875063 | 875953 | RBAM08840 | C→T (876417) | 885/885 | 294/294 |
| ABI01 1003 | 876470 | 877162 | | | 598/598 | 198/198 |
| ABI01 1286 | 1137627 | 1138262 | RBAM11610¹ | G→- (1137825) | 636/637 | 146/158 |
| ABI01_1325 | 1166675 | 1164792 | RBAM11970 | G→ -(1165370), STOP(436) | 1672/1673 | 435/435 |
| ABI01_1465 | 1281833 | 1280952 | RBAM13190 | - →G (1282256), STOP(270) | 882/882 | 269/269 |
| ABI01_1487 | 1298543 | 1299739 | RBAM13360 | 2xNt (1299663, 1299673), Q→R (374), T→K (377) | 1195/1197 | 396/398 |
| ABI01_1669 | 1500512 | 1502431 | RBAM15030 | T→G (1501024), V→G (171) | 1693/1694 | 638/639 |
| ABI01_1683 | 1519578 | 1520123 | RBAM15150 | - →A (1520100), frame shift | 545/546 | 176/177 |
| ABI01_1684 | 1520107 | 1520298 | | (177) | 192/192 | 63/63 |
| ABI01_1709 | 1543370 | 1544089 | RBAM15380 | -→GAC (1543731), T(124) | 717/720 | 238/239 |
| ABI01_1720 | 1552940 | 1551234 | RBAM15470 | T→C (1552885), S→N(17) | 1706/1707 | 567/568 |
| ABI01_1783 | 1612458 | 1612901 | RBAM16080 | G→AGA, STOP | | |
| ABI01_1785 | 1613534 | 1614877 | RBAM16100¹ | -→T, frame shift 387, STOP(400) | 1343/1344 | 388/400 |
| ABI01_2063 | 1996924 | 1997229 | RBAM18740¹ | C→A (1997886), G→-(1997994), G→- (1998023) | 1247/1250 | 347/347 |
| ABI01_2288 | 2184801 | 2184418 | RBAM20850¹ | T→A (2184169), STOP→L (121) | 383/384 | 120/120 |
| ABI01_2317 | 2207543 | 2206560 | RBAM21100² | G→A (2206731) | 983/984 | 327/327 |
| ABI01_2408 | 2292727 | 2286575 | RBAM21980² | A→G (2291591) | 6142/6143 | 2050/20 50 |
| ABI01_2702 | 2596726 | 2594366 | RBAM24730¹ | -→A (2595096), I→D (454) STOP (460) | 2360/2361 | 454/460 |
| ABI01_2731 | 2619931 | 2619647 | RBAM24990¹ | C→T (2619438) | 284/285 | 93/94 |
| ABI01_2946 | 2814887 | 2814561 | RBAM26880 | - →G(2814360), A→ - (2814363), Stop→A (85) | 326/328 | 84/84 |
| ABI01_3261 | 3113445 | 3113906 | RBAM29970 | →A (3113605), N→Q (144) | 461/462 | 143/143 |
| ABI01_3385 | 3227278 | 3225488 | RBAM31140 | T→- (3226712), - →T (3226718), R→K(100) | 1780/1782 | 595/596 |
| ABI01_3412 | 3253942 | 3252602 | RBAM31370¹ | C→- (3253028), frame shift (223) | 1341/1342 | 222/252 |
| ABI01_3565 | 3406699 | 3404525 | RBAM32850 | T→G (3405183), K→T (416) | 2174/2175 | 723/724 |
| ABI01_4037 | 3825211 | 3824033 | RBAM37130 | -→GA, R(327) | 1177/1179 | 327/327 |

| | | | | | | |
|---|---|---|---|---|---|---|
| ¹ Vermutlich Sequenzierungsfehler FZB42 ² "Stille" Mutation | | | | | | |

**Tabelle 7 Activitätsspektrum von Amylocyclicin A**

| **Indikator Stamm** | **Ergebnis^{a}** | **Quelle oder reference^{b}** |
|---|---|---|
| *Bacillus brevis* ATCC8246 | + | ATCC |
| *Bacillus subtilis* 168 | + | Stammsammlung |
| *Bacillus cereus* ATCC14579 | + | ATCC |
| *Clavibacter michiganensis* NCPPB382 | ++ | Stammsammlung |
| *Bacillus licheniformis* ATCC9789 | + | ATCC |
| *Micrococcus luteus* | ++ | Stammsammlung |
| *Bacillus pumilus* | + | Stammsammlung |
| *Bacillus subtilis* CU1065 | + | Stammsammlung |
| *Bacillus subtilis* HB0042 | ++ | Stammsammlung |
| *Bacillus sphaericus* ATCC14577 | ++ | ATCC |
| *Paenibacillus polymyxa* | + | Stammsammlung |
| *Paenibacillus granivorans* | ++ | Stammsammlung |
| *Bacillus megaterium 7A1* | + | Stammsammlung |
| *Arthrobacter spez.* | - | Stammsammlung |
| *Staphylococcus aureus* | - | Stammsammlung |
| *E. coli* K12 | - | Stammsammlung |
| *Klebsiella terrigena* | - | Stammsammlung |
| *Pseudomonas* | - | Stammsammlung |
| *Erwinia caratovora* | - | Stammsammlung |

| | | |
|---|---|---|
| ^{a} Grad der Hemmung im Biotest: ++: Starke Hemmung; +: Hemmung; -: keine Hemmung ^{b} ATCC: American Type Culture Collection | | |

**Tabelle 8: Biokontrollwirkung chemischer Fungizide (Cuprozin, Variante 3) und von Biofungiziden (FZB42, Variante 2 und ABI01, Variante 6) bei Rhizoctonia solani infizierten Kartoffeln. Die Variante 4 ist eine Kombination von FZB42 und Cuprozin). Die Anwendung 6 (ABI01) zeigt die beste Wirkung gegen Rhizoctonia solani.**

| VGL | Wdh. | Anzahl Knollen | Rhizoctonia - Pockenbesatz %* | | | | Mißbildungen | | % Rhiz |
|---|---|---|---|---|---|---|---|---|---|
| | | | 0 | < 1 | 1 bis 5 | > 5 | Anzahl | % | |
| 1 | 1 | 100 | 50 | 31 | 17 | 2 | 5 | 5,0 | |
| | 2 | 100 | 48 | 28 | 19 | 5 | 6 | 6,0 | |
| | 3 | 100 | 51 | 32 | 14 | 3 | 3 | 3,0 | |
| | 4 | 100 | 49 | 34 | 14 | 3 | 6 | 6,0 | |
| | **MW** | **100** | **49,5** | **31,3** | **16,0** | **3,3** | **5,0** | **5,0** | **24,33** |
| 2 | 1 | 100 | 64 | 32 | 3 | 1 | 4 | 4,0 | |
| | 2 | 100 | 66 | 27 | 5 | 2 | 1 | 1,0 | |
| | 3 | 100 | 63 | 34 | 3 | 0 | 3 | 3,0 | |
| | 4 | 100 | 60 | 35 | 5 | 0 | 0 | 0,0 | |
| | **MW** | **100** | **63,3** | **32,0** | **4,0** | **0,8** | **2,0** | **2,0** | **14,08** |
| 3 | 1 | 100 | 64 | 28 | 7 | 1 | 3 | 3,0 | |
| | 2 | 100 | 61 | 33 | 6 | 0 | 5 | 5,0 | |
| | 3 | 100 | 64 | 26 | 10 | 0 | 4 | 4,0 | |
| | 4 | 100 | 66 | 25 | 8 | 1 | 4 | 4,0 | |
| | **MW** | **100** | **63,8** | **28,0** | **7,8** | **0,5** | **4,0** | **4,0** | **15,00** |
| 4 | 1 | 100 | 54 | 35 | 11 | 0 | 3 | 3,0 | |
| | 2 | 100 | 59 | 29 | 9 | 3 | 2 | 2,0 | |
| | 3 | 100 | 59 | 29 | 12 | 0 | 5 | 5,0 | |
| | 4 | 100 | 53 | 33 | 13 | 1 | 3 | 3,0 | |
| | **MW** | **100** | **56,3** | **31,5** | **11,3** | **1,0** | **3,3** | **3,3** | **19,00** |
| 6 | 1 | 100 | 79 | 16 | 5 | 0 | 0 | 0,0 | |
| | 2 | 100 | 76 | 15 | 7 | 2 | 2 | 2,0 | |
| | 3 | 100 | 80 | 15 | 3 | 2 | 0 | 0,0 | |
| | 4 | 100 | 73 | 17 | 8 | 2 | 1 | 1,0 | |
| | **MW** | **100** | **77,0** | **15,8** | **5,8** | **1,5** | **0,8** | **0,8** | **10,58** |

| | | | | | | | | | |
|---|---|---|---|---|---|---|---|---|---|
| * von 100 Knollen aus der Fraktion 35 - 55 mm | | | | | | | | | |

**Tabelle 9. Befall von Tabakpflanzen mit TMV (Tabak-Mosaik-Virus), bakterieller Welke (Ralstonia solanacearum), Braunfleckenkrankheit (Alternaria alternata) und echter Mehltau (Erysiphe cichoracearum). Varianten: 1 (Kontrolle, Wasser), 2 , 3 (Bodenverbesserer), 4, 5 (FZB42).**

| **Zeit** | **Variante** | TMV (Tabakmosaikvirus) | | Tabak bakterielle Welke | | Braunfleckenkrankheit | | Echter Mehltau | | Tabak "Climatic spot" | |
|---|---|---|---|---|---|---|---|---|---|---|---|
| | | Krankheitsindex | Inzidenz | Krankheitsindex | Inzidenz | Krankheitsindex | Inzidenz | Krankheitsindex | Inzidenz | Krankhei tsindex | Inzidenz |
| Jun. 29 | 1 | 27.78a | 4.57a | 9.80 | 1.96 | 23.53 | 1.31 | 43.14 | 3.27 | 7.84 | 0.65 |
| | 2 | 6.53b | 1.31b | 3.27 | 0.65 | 11.76 | 0.65 | 31.37 | 1.96 | 0.00 | 0.00 |
| | 3 | 8.17b | 1.31b | 3.27 | 0.65 | 11.76 | 0.65 | 15.69 | 1.31 | 0.00 | 0.00 |
| | 4 | 3.27b | 0.65b | 0.00 | 0.00 | 0.00 | 0.00 | 6.54 | 0.65 | 0.00 | 0.00 |
| | 5 | 6.53b | 1.31b | 0.00 | 0.00 | 11.76 | 0.65 | 9.15 | 0.65 | 0.00 | 0.00 |
| July 16 | 1 | 42.48b | 6.53b | 19.61a | 3.92a | 62.75 | 2.61 | 94.12 | 5.23 | 43.14 | 2.61 |
| | 2 | 22.87ab | 4.57ab | 3.27b | 0.65b | 47.06 | 1.96 | 66.67 | 3.92 | 23.53 | 1.96 |
| | 3 | 22.88ab | 3.27ab | 9.80ab | 1.96ab | 47.06 | 1.96 | 47.06 | 2.61 | 15.69 | 1.31 |
| | 4 | 9.80a | 1.96a | 3.27b | 0.65b | 15.69 | 0.65 | 27.45 | 1.96 | 27.45 | 1.96 |
| | 5 | 19.61ab | 3.92ab | 3.27b | 0.65b | 31.37 | 1.31 | 27.45 | 1.31 | 32.68 | 1.96 |
| Aug. 2 | 1 | 84.97a | 8.49a | 29.41a | 5.88a | 105.88 | 4.57 | 117.65 | 6.53 | 117.65 | 6.53 |
| | 2 | 45.75ab | 5.23ab | 9.80b | 1.96b | 78.43 | 3.27 | 66.67 | 3.92 | 82.35 | 4.57 |
| | 3 | 52.29ab | 5.23ab | 13.07ab | 2.61ab | 43.14 | 1.96 | 70.59 | 3.92 | 62.75 | 3.92 |
| | 4 | 16.34b | 3.27b | 9.80b | 1.96b | 31.37 | 1.31 | 52.94 | 3.27 | 52.94 | 3.27 |
| | 5 | 35.95ab | 5.23b | 9.80b | 1.96b | 45.10 | 1.96 | 56.86 | 3.27 | 72.55 | 3.92 |

## Patentansprüche

1. Mittel zur Behandlung von pilzlichen Erkrankungen bei Kulturpflanzen, **dadurch gekennzeichnet, dass** es den Stamm Bacillus amyloliquefaciens subsp. plantarum ABI01, DSM 10-1092 enthält.

2. Mittel nach Anspruch 1, **dadurch gekennzeichnet, dass** es eine flüssige Sporensuspension enthält, die bevorzugt mindestens 1 x 10¹⁰ Sporen/ml enthält.

3. Mittel nach Anspruch 1, **dadurch gekennzeichnet, dass** es ein Trockenpräparat ("Trockenbeize") enthält, welches bevorzugt mindestens 2 x 10¹⁰ Sporen/g enthält.

4. Mittel nach Anspruch 1, **dadurch gekennzeichnet, dass** es ein Flüssig-Konzentrat, gewonnen aus einer Bacillus amyloliquefaciens subsp. plantarum ABI01 Kultur, mit einer Lipopeptidkonzentration von mindestens 1 g/L enthält.

5. Mittel nach Anspruch 1, **dadurch gekennzeichnet, dass** es ein Flüssig-Konzentrat, gewonnen aus einer Bacillus amyloliquefaciens subsp. plantarum ABI01 Kultur, mit einer Bacillomycin D Konzentration von mindestens 1 g/L enthält.

6. Mittel nach Anspruch 1, **dadurch gekennzeichnet, dass** es ein Flüssig-Konzentrat, gewonnen aus einer Bacillus amyloliquefaciens subsp. plantarum ABI01 Kultur, mit einer Lipopeptidkonzentration von mindestens 1 g/L, und einem Sporengehalt von mindestens 1 x 10¹⁰ Sporen/ g (ml) enthält.

7. Mittel nach Anspruch 1, **dadurch gekennzeichnet, dass** es ein Flüssig-Konzentrat, gewonnen aus einer Bacillus amyloliquefaciens subsp. plantarum ABI01 Kultur, mit einer Bacillomycin D Konzentration von mindestens 1 g/L, und einem Sporengehalt von mindestens 1 x 10¹⁰ Sporen/g (ml) enthält.

8. Verwendung der Mittel nach einem der Ansprüche 1 bis 3 als Gießpräparate während der Pflanzung.

9. Verwendung der Mittel nach einem der Ansprüche 1 und 4 bis 7 als Blattsprühmittel.

10. Bacillus amyloliquefaciens subsp. plantarum-Stamm, ABI01, DSM 10-1092 in einem Mittel gemäß einem der Ansprüche 1-7, **gekennzeichnet durch** die gleichzeitige Anwesenheit der folgenden Gencluster: Gencluster für die ribosomale Synthese eines neuen, antimikrobiellen, zyklischen Peptids "Amylocyclicin" (1), Gencluster für die ribosomale Synthese des antimikrobiellen, Oxazol/Thiazol enthaltenden Peptids "Plantazolicin" (2), Gencluster für die nichtribosomale Synthese eines antimikrobiellen Lipopeptids "Surfactin" (3), Gencluster für die nichtribosomale Synthese eines antifungalen Lipopeptids der Ituringruppe, z.B. Bacillomycin D (4), Gencluster für die nichtribosomale Synthese eines antifungalen Lipopeptids der Fengycin/Plipastatingruppe, z.B. Fengycin (5), Gencluster für die nicht-ribosomale Synthese eines Fe-Siderophors, z.B. Bacillibactin (6), Gencluster für die nicht-ribosomale Synthese der antibakteriell wirkenden Polyketide Makrolaktin (7), Bacillaen (8), und Difficidin (9) und die nicht-ribosomale Synthese des antimikrobiellen Dipeptids Bacilysin (10).

11. Bacillus amyloliquefaciens subsp. plantarum-Stamm ABI01, DSM 10-1092 in einem Mittel gemäß einem der Ansprüche 1-7, **gekennzeichnet durch** die Fähigkeit
- zur lang andauernden, insbesondere mehr als vier Monate, kompetitiven Besiedlung der pflanzlichen Wurzelzone,
- zur Unterdrückung phytopathogener Mikroorganismen,
- zur Unterdrückung phytopathogener Pilze,
- zur Unterdrückung des phytopathogenen Pilzes Rhizoctonia solani,
- zur Unterdrückung phytopathogener Bakterien,
- zur Unterdrückung phytopathogener Viren,
- zur Unterdrückung von Tabak-Mosaik-Virus (TMV),
- zur Unterdrückung phytopathogene Fadenwürmer (Nematoden).

## Claims

1. An agent for treating fungal diseases in cultivated plants, **characterized in that** it contains the strain bacillus amyloliquefaciens subsp. plantarum ABI01 (DSM 10-1092).

2. The agent according to Claim 1, **characterized in that** it contains a liquid spore suspension, which preferably contains at least 1 x 10¹⁰ spores/ml.

3. The agent according to Claim 1, **characterized in that** it contains a dry preparation ("dry powder"), which preferably contains at least 2 x 10¹⁰ spores/g.

4. The agent according to Claim 1, **characterized in that** it contains a liquid concentrate obtained from a bacillus amyloliquefaciens subsp. plantarum ABI01 culture, with a lipopeptide concentration of at least 1 g/l.

5. The agent according to Claim 1, **characterized in that** it contains a liquid concentrate obtained from a bacillus amyloliquefaciens subsp. plantarum ABI01 culture, with a bacillomycin D concentration of at least 1 g/l.

6. The agent according to Claim 1, **characterized in that** it contains a liquid concentrate obtained from a bacillus amyloliquefaciens subsp. plantarum ABI01 culture, with a lipopeptide concentration of at least 1 g/l and a spore content of at least 1 x 10¹⁰ spores/g (ml).

7. The agent according to Claim 1, **characterized in that** it contains a liquid concentrate obtained from a bacillus amyloliquefaciens subsp. plantarum ABI01 culture, with a bacillomycin D concentration of at least 1 g/l and a spore content of at least 1 x 10¹⁰ spores/g (ml).

8. The use of the agents according to any one of Claims 1 to 3 as pourable preparations during planting.

9. The use of the agents according to any one of Claims 1 and 4 to 7 as a leaf-spraying agent.

10. Bacillus amyloliquefaciens subsp. plantarum strain, ABI01 (DSM 10-1092) in an agent according to any one of Claims 1-7, **characterized by** the simultaneous presence of the following gene clusters: gene cluster for the ribosomal synthesis of a new, antimicrobial, cyclical peptide "amylocyclicin" (1), gene cluster for the ribosomal synthesis of the antimicrobial peptide "plantazolicin" (2) containing oxazol/thiazol, gene cluster for the non-ribosomal synthesis of an antimicrobial lipopeptide "surfactin" (3), gene cluster for the non-ribosomal synthesis of an anti-fungal lipopeptide from the iturin group, e.g. bacillomycin D (4), gene cluster for the non-ribosomal synthesis of an anti-fungal lipopeptide of the fengycin/plipastatin group, e.g. fengycin (5), gene cluster for the non-ribosomal synthesis of an Fe-siderophor, e.g. bacillibactin (6), gene cluster for the non-ribosomal synthesis of the anti-bacterial polyketide macrolactin (7), bacillae (8), and difficidin (9) and the non-ribosomal synthesis of the anti-microbial dipeptide bacilysin (10).

11. Bacillus amyloliquefaciens subsp. plantarum strain, ABI01 (DSM 10-1092) in an agent according to any one of Claims 1-7, **characterized by** the ability
- to be long-lasting, in particular lasting more than four months, competitive colonisation of the plant root zone,
- to suppress phytopathogenic micro-organisms,
- to suppress phytopathogenic fungi,
- to suppress the phytopathogenic fungus rhizoctonia solani,
- to suppress phytopathogenic bacteria,
- to suppress phytopathogenic viruses,
- to suppress the tobacco mosaic virus (TMV),
- to suppress phytopathogenic threadworms (nematodes).

## Revendications

1. Agent destiné au traitement des maladies fongiques dans les cultures, **caractérisé en ce qu'**il contient la souche Bacillus amyloliquefaciens subsp. plantarum ABI01 (DSM 10-1092).

2. Agent conformément à la revendication n°1, **caractérisé en ce qu'**il contient une suspension de spores liquide, contenant de préférence au moins 1 x 10¹⁰ spores/ml.

3. Agent conformément à la revendication n°1, **caractérisé en ce qu'**il contient une préparation sèche (« décapant à sec »), contenant de préférence au moins 2 x 10¹⁰ spores/g.

4. Agent conformément à la revendication n°1, **caractérisé en ce qu'**il contient un concentré liquide à base d'une culture de Bacillus amyloliquefaciens subsp. plantarum ABI01, avec une concentration en lipopeptides d'au moins 1 g/l.

5. Agent conformément à la revendication n°1, **caractérisé en ce qu'**il contient un concentré liquide à base d'une culture de Bacillus amyloliquefaciens subsp. plantarum ABI01, avec une concentration en bacillomycine D d'au moins 1 g/l.

6. Agent conformément à la revendication n°1, **caractérisé en ce qu'**il contient un concentré liquide à base d'une culture de Bacillus amyloliquefaciens subsp. plantarum ABI01, avec une concentration en lipopeptides d'au moins 1 g/l et une teneur en spores de minimum 1 x 10¹⁰ spores/g (ml).

7. Agent conformément à la revendication n°1, **caractérisé en ce qu'**il contient un concentré liquide à base d'une culture de Bacillus amyloliquefaciens subsp. plantarum ABI01, avec une concentration en bacillomycine D d'au moins 1 g/l et une teneur en spores de minimum 1 x 10¹⁰ spores/g (ml).

8. Utilisation des agents conformément à l'une des revendications n°1 à n°3 comme préparations à verser pendant la plantation.

9. Utilisation des agents conformément à l'une des revendications n°1 et n°4 à n°7 comme agents à vaporiser sur les feuilles.

10. Souche Bacillus amyloliquefaciens subsp. plantarum, ABI01 (DSM 10-1092) dans un agent conformément à l'une des revendications n°1 à n°7, **caractérisé par** la présence simultanée des groupements de gènes suivants : groupement de gènes pour la synthèse ribosomale d'un nouveau peptide cyclique, antimicrobien « amylocyclicine » (1), groupement de gènes pour la synthèse ribosomale du peptide antimicrobien, contenant de l'oxazole/du thiazole « plantazolicine » (2), groupement de gènes pour la synthèse non-ribosomale d'un lipppeptide antimicrobien « surfactine » (3), groupement de gènes pour la synthèse non-ribosomale d'un lipppeptide antifongique du groupe des iturines, par ex. bacillomycine D (4), groupement de gènes pour la synthèse non-ribosomale d'un lipppeptide antifongique du groupe des fengycines/plipastatines, par ex. fengycine (5), groupement de gènes pour la synthèse non-ribosomale d'un sidérophore Fe, par ex. bacillibactine (6), groupement de gènes pour la synthèse non-ribosomale des polycétides à action antibactérienne, macrolactine (7), bacillaène (8), et difficidine (9) et la synthèse non-ribosomale du dipeptide antimicrobien bacilysine (10).

11. Souche Bacillus amyloliquefaciens subsp. plantarum ABI01 (DSM 10-1092) dans un agent conformément à l'une des revendications n°1 à n°7, **caractérisé par** la capacité
- de colonisation compétitive de la zone des racines des plantes pendant une longue durée, notamment plus de quatre mois,
- d'inhibition de microorganismes phytopathogènes,
- d'inhibition de champignons phytopathogènes,
- d'inhibition du champignon phytopathogène Rhizoctonia solani,
- d'inhibition de bactéries phytopathogènes,
- d'inhibition de virus phytopathogènes,
- d'inhibition du virus de la mosaïque du tabac (TMV),
- d'inhibition de nématodes phytopathogènes.
